# EUROPEAN PATENT APPLICATION

(11) **EP 3 960 802 A1**
(43) Date of publication of application: **02.03.2022**
(21) Application number: 20795102.1
(22) Date of filing: 24.04.2020
(51) Int. Cl.: C08K 5/101, C08K 5/14, C08L 23/02

(54) **POLYOLEFIN RESIN COMPOSITION AND PRODUCTION METHOD THEREOF**

(30) Priority: 25.04.2019 JP 2019084196
(71) Applicant: SUMITOMO CHEMICAL COMPANY LIMITED, Chuo-ku Tokyo 104-8260 (JP)
(72) Inventor: KIMURA, Natsuko, Osaka-shi, Osaka 554-8558 (JP); HIGUCHI, Yu, Ichihara-shi, Chiba 299-0195 (JP); MIYATAKE, Toshiaki, Niihama-shi, Ehime 792-8521 (JP)
(74) Representative: Winter, Brandl - Partnerschaft mbB
(86) International application number: PCT/JP2020/017828
(87) International publication number: WO 2020/218574

(57) **Abstract**

The present invention relates to a polyolefin resin composition containing at least: (A) 100 parts by mass of a polyolefin resin, (B) 0.01 to 5 parts by mass of a compound having at least one (meth)acrylate group, and one hydrogen bonding group or one (meth)acrylate group, and (C) 0.01 to 5 parts by mass of an organic peroxide.

## Description

### Technical Field

The present invention relates to a polyolefin resin composition and a method for producing the same.

### Background Art

Polyolefin resins are used as materials for various industrial parts, for example, interior and exterior parts such as bumpers, instrument panels, door trims, and pillars in automobile applications, and parts of vacuum cleaners, televisions, and the like in household applications. These parts are required to have rigidity and impact resistance, but due to the recent increasing demand for weight reduction of automobiles and household electrical appliances, weight reduction is also demanded in parts to be used in automobiles and household electrical appliances.

As one method for reducing the weight of parts, a method for reducing the thickness of parts is considered, but the reduction in thickness may lower impact resistance. When parts with a shape having a reduced thickness are molded, an improvement in the fluidity of a polyolefin resin that constitutes the parts is needed, but the improvement in the fluidity of a resin may also lower impact resistance.

With respect to a composition including a polyolefin resin, for example, Patent Literature 1 discloses a resin composition including a specific propylene polymer, and a copolymer of ethylene and either propylene or an α-olefin having 4 to 20 carbon atoms. Patent Literature 2 discloses a thermoplastic polymer composition including a thermoplastic polymer and a specific compatibilizer.

### Citation List

### Patent Literature

Patent Literature 1: Japanese Patent Laid-Open No. 2013-79375
Patent Literature 2: National Publication of International Patent Application No. 2018-505284

### Summary of Invention

### Technical Problem

Although various resin compositions as described above have been investigated, there is still a demand for improving the impact resistance and fluidity of the polyolefin resin composition. Furthermore, the polyolefin resin composition is used without coloring or optionally with coloring depending on the characteristics required for parts, and in both cases, the color tone derived from the polyolefin resin composition before coloring (e.g. yellowness) is desirably reduced.

Therefore, an object of the present invention is to provide a resin composition having a reduced color tone derived from the polyolefin resin composition itself and excellent impact resistance and fluidity.

### Solution to Problem

The present inventors have intensively studied to solve the above problems by focusing on kinds and amount of components contained in the resin composition. As a result, the present inventors have found that a polyolefin resin composition containing specific components can solve the above problems, and have achieved the present invention.

That is, the present invention encompasses the following preferred aspects.
[1] A polyolefin resin composition comprising at least:
   (A) 100 parts by mass of a polyolefin resin,
   (B) 0.01 to 5 parts by mass of a compound having at least one (meth)acrylate group, and one hydrogen bonding group or one (meth)acrylate group, and
   (C) 0.01 to 5 parts by mass of an organic peroxide.
[2] The polyolefin resin composition according to the item [1], wherein (B) the compound having at least one (meth)acrylate group, and one hydrogen bonding group or one (meth)acrylate group is represented by formula (I) :
   [Formula 1] 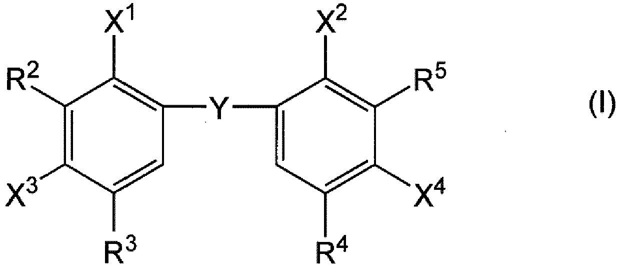 wherein
   X¹ and X² each independently represent a hydrogen bonding group or a group represented by -OC(=O)CR¹=CH₂ and X³ and X⁴ each independently represent a hydrogen atom or a linear or branched alkyl group having 1 to 9 carbon atoms, or X¹ and X² each independently represent a hydrogen atom or a linear or branched alkyl group having 1 to 9 carbon atoms and X³ and X⁴ each independently represent a hydrogen bonding group or a group represented by -OC(=O)CR¹=CH₂, provided that at least one of X¹ to X⁴ represents a group represented by -OC(=O)CR¹=CH₂, and R¹ represents a hydrogen atom or a methyl group;
   Y represents a single bond, a sulfide bond, or a linear or branched alkylene group having 1 to 9 carbon atoms; and
   R² and R⁵ each independently represent a hydrogen atom or a linear or branched alkyl group having 1 to 9 carbon atoms, and R³ and R⁴ each independently represent a linear or branched alkyl group having 1 to 9 carbon atoms.
[3] The polyolefin resin composition according to the item [2], wherein in formula (I), X¹ represents a hydrogen bonding group or a group represented by - OC (=O)CR¹=CH₂; X² represents a group represented by - OC (=O) CR¹=CH₂; X³ and X⁴ represent a hydrogen atom; Y represents a single bond or a linear or branched alkylene group having 1 to 9 carbon atoms; R¹ represents a hydrogen atom or a methyl group; and R² to R⁵ each independently represent a linear or branched alkyl group having 1 to 9 carbon atoms.
[4] The polyolefin resin composition according to the item [2], wherein in formula (I), X¹ and X² represent a linear or branched alkyl group having 1 to 9 carbon atoms; X³ represents a hydrogen bonding group or a group represented by -OC(=O)CR¹=CH₂; X⁴ represents a group represented by -OC(=O)CR¹=CH₂; Y represents a sulfide bond; R¹ represents a hydrogen atom or a methyl group; R² and R⁵ represent a hydrogen atom; and R³ and R⁴ each independently represent a linear or branched alkyl group having 1 to 9 carbon atoms.
[5] The polyolefin resin composition according to the item [1], wherein (B) the compound having at least one (meth)acrylate group, and one hydrogen bonding group or one (meth)acrylate group is a compound having one (meth)acrylate group and at least one hydrogen bonding group.
[6] The polyolefin resin composition according to the item [5], wherein (B) the compound having one (meth)acrylate group and at least one hydrogen bonding group is represented by formula (Ia):
   [Formula 2] 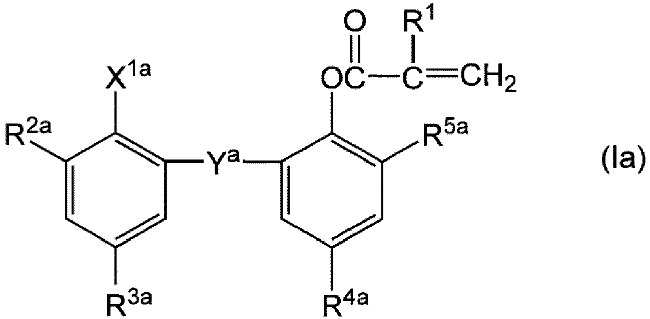 wherein
   X^{1a} represents a hydrogen bonding group;
   Y^{a} represents a single bond or a linear or branched alkylene group having 1 to 9 carbon atoms;
   R¹ represents a hydrogen atom or a methyl group; and
   R^{2a} to R^{5a} each independently represent a linear or branched alkyl group having 1 to 9 carbon atoms.
[7] The polyolefin resin composition according to the item [6], wherein formula (I) is represented by formula (Ia'):
   [Formula 3] 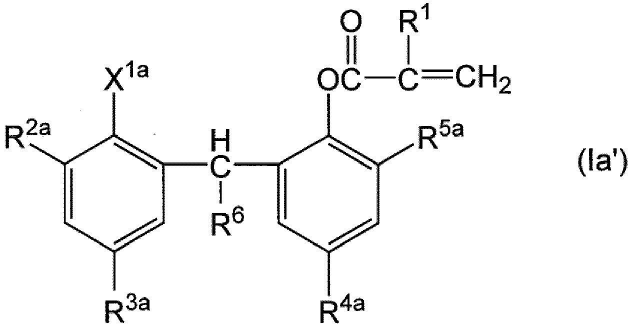 wherein X^{1a}, R¹ and R^{2a} to R^{5a} are as defined for X^{1a}, R¹ and R^{2a} to R^{5a} in formula (Ia), respectively; and R⁶ represents a hydrogen atom or a methyl group.
[8] The polyolefin resin composition according to any one of the items [1] to [7], wherein the hydrogen bonding group is selected from the group consisting of a hydroxyl group, an amino group, a thiol group, a carboxyl group, a sulfonic acid group, and a phosphoric acid group.
[9] The polyolefin resin composition according to the item [7], wherein in formula (Ia'), X^{1a} represents a hydroxyl group; R¹ represents a hydrogen atom; R^{2a} to R^{5a} represent a 1,1-dimethylpropyl group; and R⁶ represents a methyl group.
[10] The polyolefin resin composition according to the item [7], wherein in formula (Ia'), X^{1a} represents a hydroxyl group; R¹ and R⁶ represent a hydrogen atom; R^{2a} and R^{5a} represent a t-butyl group; R^{3a} and R^{4a} represent a methyl group.
[11] The polyolefin resin composition according to the item [2], wherein formula (I) is represented by formula (1d):
   [Formula 4] 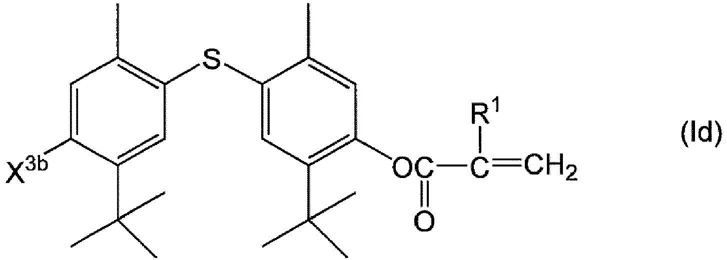 wherein X^{3b} represents a hydrogen bonding group or a group represented by -OC(=O)CR¹=CH₂; and R¹ represents a hydrogen atom.
[12] The polyolefin resin composition according to any one of the items [1] to [11], wherein (C) the organic peroxide is selected from the group consisting of an alkyl peroxide compound, a diacyl peroxide compound, a peroxy ester compound, and a peroxycarbonate compound.
[13] The polyolefin resin composition according to any one of the items [1] to [12], wherein a mass ratio of (B) the compound having at least one (meth)acrylate group, and one hydrogen bonding group or one (meth)acrylate group:(C) the organic peroxide is 1:10 to 10:1.
[14] The polyolefin resin composition according to any one of the items [1] to [13], wherein (A) the polyolefin resin comprises a propylene-based resin.
[15] The polyolefin resin composition according to the item [14], wherein the propylene-based resin comprises 80% by mass or more of a monomer unit derived from propylene, based on the total amount of the propylene-based resin.
[16] The polyolefin resin composition according to the item [14] or [15], wherein (A) the polyolefin resin further comprises an ethylene-based copolymer.
[17] The polyolefin resin composition according to the item [16], wherein the ethylene-based copolymer comprises 20% by mass or more of a monomer unit derived from ethylene, based on the total amount of the ethylene-based copolymer.
[18] A method for producing the polyolefin resin composition according to any one of the items [1] to [17], comprising at least a step of mixing (A) a polyolefin resin, (B) a compound having at least one (meth)acrylate group, and one hydrogen bonding group or one (meth)acrylate group, and (C) an organic peroxide together by melt-kneading a mixture obtained by dry-mixing in advance.
[19] The production method according to the item [18], comprising either step (1) of dry-mixing (A) the polyolefin resin, (B) the compound having at least one (meth)acrylate group, and one hydrogen bonding group or one (meth)acrylate group, and (C) the organic peroxide to give a mixture; and step (2) of melt-kneading the mixture obtained in step (1) to give the polyolefin resin composition, or
   step (1a) of dry-mixing (A) the polyolefin resin and a part of (C) the organic peroxide to give a first dry mixture, step (2a) of melt-kneading the dry mixture obtained in step (1a) to give a first premixture; step (1b) of dry-mixing the first premixture obtained in step (2a), (B) the compound having at least one (meth)acrylate group, and one hydrogen bonding group or one (meth)acrylate group, and another part of (C) the organic peroxide to give a second dry mixture; and step (2b) of melt-kneading the dry mixture obtained in step (1b) to give the polyolefin resin composition.
[20] The production method according to the item [18] or [19], wherein the mixing of (B) the compound having at least one (meth)acrylate group, and one hydrogen bonding group or one (meth)acrylate group in step (1) and step (1b) is carried out by using a masterbatch comprising at least the compound and at least one resin selected from the group consisting of a polyolefin-based resin and an ethylene-based copolymer.
[21] A masterbatch comprising a compound having at least one (meth)acrylate group, and one hydrogen bonding group or one (meth)acrylate group, and at least one resin selected from the group consisting of a polyolefin-based resin and an ethylene-based copolymer.
[22] The masterbatch according to the item [21], comprising 10 to 50 parts by mass of the compound having at least one (meth)acrylate group, and one hydrogen bonding group or one (meth)acrylate group with respect to 100 parts by mass of the polyolefin-based resin and the ethylene-based copolymer.
[23] The masterbatch according to the item [20] or [21], further comprising an organic peroxide.
[24] A compound represented by formula (Ic):
   [Formula 5] 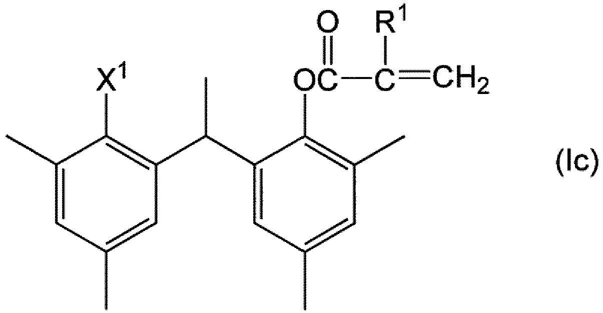 wherein X¹ represents a hydrogen bonding group or a group represented by -OC(=O)CR¹=CH₂; and R¹ represents a hydrogen atom.
[25] A compound represented by formula (Id):
   [Formula 6] 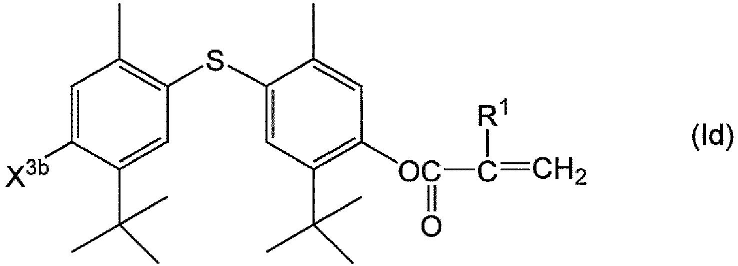 wherein X^{3b} represents a hydrogen bonding group or a group represented by -OC(=O)CR¹=CH₂; and R¹ represents a hydrogen atom.

The present invention also encompasses the following aspects in a preferred aspect.
[1'] A polyolefin resin composition comprising at least:
   (A) 100 parts by mass of a polyolefin resin;
   (B) 0.01 to 5 parts by mass of a compound having one (meth)acrylate group and at least one hydrogen bonding group; and
   (C) 0.01 to 5 parts by mass of an organic peroxide.
[2'] The polyolefin resin composition according to the item [1'], wherein (B) the compound having one (meth)acrylate group and at least one hydrogen bonding group is represented by formula (Ia):
   [Formula 7] 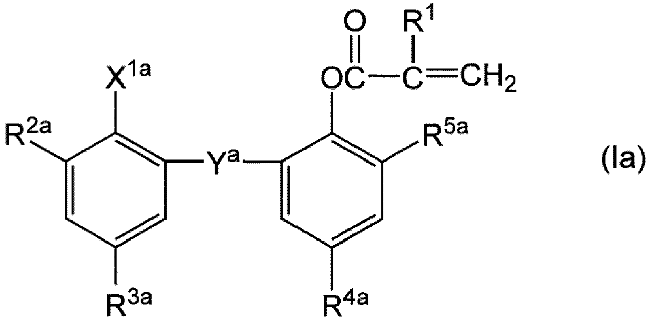 wherein
   X^{1a} represents a hydrogen bonding group;
   Y^{a} represents a single bond or a linear or branched alkylene group having 1 to 9 carbon atoms;
   R¹ represents a hydrogen atom or a methyl group; and
   R^{2a} to R^{5a} each independently represent a linear or branched alkyl group having 1 to 9 carbon atoms.
[3'] The polyolefin resin composition according to the item [2'], wherein formula (I) is represented by formula (Ia'):
   [Formula 8] 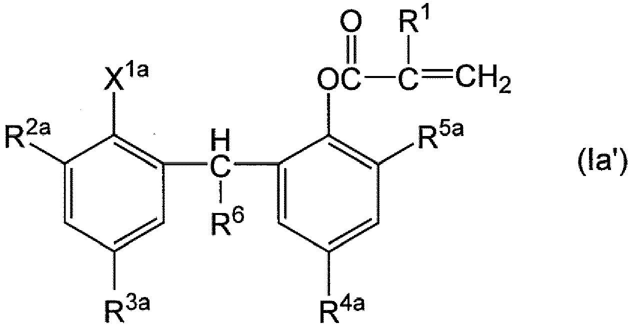 wherein X^{1a}, R¹ and R^{2a} to R^{5a} are as defined for X^{1a}, R¹ and R^{2a} to R^{5a} in formula (Ia), respectively; and R⁶ represents a hydrogen atom or a methyl group.
[4'] The polyolefin resin composition according to any one of the items [1'] to [3'], wherein the hydrogen bonding group is selected from the group consisting of a hydroxyl group, an amino group, a thiol group, a carboxyl group, a sulfonic acid group, and a phosphoric acid group.
[5'] The polyolefin resin composition according to the item [3'], wherein in formula (Ia'), X^{1a} represents a hydroxyl group; R¹ represents a hydrogen atom; R^{2a} to R^{5a} represent a 1,1-dimethylpropyl group; and R⁶ represents a methyl group.
[6'] The polyolefin resin composition according to the item [3'], wherein in formula (Ia'), X^{1a} represents a hydroxyl group; R¹ and R⁶ represent a hydrogen atom; R^{2a} and R^{5a} represent a t-butyl group; R^{3a} and R^{4a} represent a methyl group.
[7'] The polyolefin resin composition according to any one of the items [1'] to [6'], wherein (C) the organic peroxide is selected from the group consisting of an alkyl peroxide compound, a diacyl peroxide compound, a peroxy ester compound, and a peroxycarbonate compound.
[8'] The polyolefin resin composition according to any one of the items [1'] to [7'], wherein a mass ratio of (B) the compound having one (meth)acrylate group and at least one hydrogen bonding group:(C) the organic peroxide is 1:10 to 10:1.
[9'] The polyolefin resin composition according to any one of the items [1'] to [8'], wherein (A) the polyolefin resin comprises a propylene-based resin.
[10'] The polyolefin resin composition according to the item [9'], wherein the propylene-based resin comprises 80% by mass or more of a monomer unit derived from propylene, based on the total amount of the propylene-based resin.
[11'] The polyolefin resin composition according to the item [9'] or [10'], wherein (A) the polyolefin resin further comprises an ethylene-based copolymer.
[12'] The polyolefin resin composition according to the item [11'], wherein the ethylene-based copolymer comprises 20% by mass or more of a monomer unit derived from ethylene, based on the total amount of the ethylene-based copolymer.
[13'] A method for producing the polyolefin resin composition according to any one of the items [1'] to [12'], comprising at least a step of mixing (A) a polyolefin resin, (B) a compound having one (meth)acrylate group and at least one hydrogen bonding group, and (C) an organic peroxide together by melt-kneading a mixture obtained by dry-mixing in advance.
[14'] The production method according to the item [13'], comprising either step (1) of dry-mixing (A) the polyolefin resin, (B) the compound having one (meth)acrylate group and at least one hydrogen bonding group, and (C) the organic peroxide to give a mixture; and step (2) of melt-kneading the mixture obtained in step (1) to give the polyolefin resin composition, or
   step (1a) of dry-mixing (A) the polyolefin resin and a part of (C) the organic peroxide to give a first dry mixture; step (2a) of melt-kneading the dry mixture obtained in step (1a) to give a first premixture; step (1b) of dry-mixing the first premixture obtained in step (2a), (B) the compound having one (meth)acrylate group and at least one hydrogen bonding group, and another part of (C) the organic peroxide to give a second dry mixture; and step (2b) of melt-kneading the dry mixture obtained in step (1b) to give the polyolefin resin composition.

### Advantageous Effect of Invention

According to the present invention, a polyolefin resin composition having a reduced color tone derived from the polyolefin resin composition itself and excellent impact resistance and fluidity is provided.

### Description of Embodiments

Hereinafter, the embodiments of the present invention will be described in detail. The scope of the present invention is not limited to the embodiments described herein and can be variously changed within a range not departing from the spirit of the present invention.

### <Polyolefin Resin Composition>

The polyolefin resin composition of the present invention contains at least: (A) 100 parts by mass of a polyolefin resin, (B) 0.01 to 5 parts by mass of a compound having at least one (meth)acrylate group, and one hydrogen bonding group or one (meth)acrylate group, and (C) 0.01 to 5 parts by mass of an organic peroxide. The polyolefin resin composition of the present invention contains the polyolefin resin as the main component, as described above, 0.01 to 5 parts by mass of (B) the compound having at least one (meth)acrylate group, and one hydrogen bonding group or one (meth)acrylate group with respect to 100 parts by mass of the polyolefin resin, and 0.01 to 5 parts by mass of (C) the organic peroxide with respect to 100 parts by mass of the polyolefin resin. In the present specification, a compound having at least one (meth)acrylate group, and one hydrogen bonding group or one (meth)acrylate group is also referred to as the "compound (B)".

In one aspect of the present invention, the polyolefin resin composition of the present invention contains at least: (A) 100 parts by mass of a polyolefin resin, (B) 0.01 to 5 parts by mass of a compound having one (meth)acrylate group and at least one hydrogen bonding group, and (C) 0.01 to 5 parts by mass of an organic peroxide. The polyolefin resin composition of the present invention contains the polyolefin resin as the main component, as described above, 0.01 to 5 parts by mass of (B) the compound having one (meth)acrylate group and at least one hydrogen bonding group with respect to 100 parts by mass of the polyolefin resin, and 0.01 to 5 parts by mass of (C) the organic peroxide with respect to 100 parts by mass of the polyolefin resin. In the present specification, the compound having one (meth)acrylate group and at least one hydrogen bonding group is also referred to as the "compound (Ba)". Unless otherwise indicated, the description about the following compound (B) also applies to other compounds (Ba) and the like.

In the polyolefin resin composition of the present invention, the reason why a color tone derived from the polyolefin resin composition itself is reduced and impact resistance and fluidity are improved is not clear, but this is considered to be likely due to the following mechanism. The present invention is not limited to the following mechanism in any way. When an organic peroxide is contained in a polyolefin resin composition, the organic peroxide is considered to have the functions of decomposing the polyolefin resin and improving the fluidity of the resin. On the other hand, the compound (B) contained in the polyolefin resin composition, since having one (meth)acrylate group, and one hydrogen bonding group or one (meth)acrylate group, reacts with the organic peroxide, as well as a (meth)acrylate group or a hydrogen bonding group in the compound (B) moderately reacts and/or interacts with a polyolefin resin and interacts with a (meth)acrylate group or a hydrogen bonding group of the compound (B) which is interacting with another polyolefin resin. It is considered that as a result, the compound (B) has functions of compatibilizing, for example, a propylene-based resin and an ethylene-based copolymer in the polyolefin resin, finely dispersing the propylene-based resin and the ethylene-based copolymer, and thereby improving the impact resistance of the polyolefin resin composition.

In particular, when the compound (B) is the compound (Ba) having one (meth)acrylate group and at least one hydrogen bonding group, the compound (Ba), since having one (meth)acrylate group and at least one hydrogen bonding group, reacts with the organic peroxide, as well as the (meth)acrylate group of the compound (Ba) moderately reacts and/or interacts with the polyolefin resin. Further, the hydrogen bonding group of the compound (Ba) forms a hydrogen bond with a hydrogen bonding group of another compound (Ba) which is interacting with a polyolefin resin, so that, for example, a propylene-based resin and an ethylene-based copolymer in the polyolefin resin are compatibilized. It is considered that as a result, the compound (Ba) has functions of finely dispersing the propylene-based resin and the ethylene-based copolymer, and improving the impact resistance of the polyolefin resin composition. Surprisingly, it is found that molded bodies obtained from the polyolefin resin composition of the present invention having specific compound (B) and organic peroxide has low yellowness.

In the polyolefin resin composition of the present invention, the content of the compound (B) is 0.01 to 5 parts by mass with respect to (A) 100 parts by mass of a polyolefin resin. When the content of the compound (B) is less than 0.01 parts by mass, the polyolefin resin is considered to be not sufficiently finely dispersed and the impact resistance of the molded body to be obtained cannot be sufficiently increased. In contrast, when the content of the compound (B) is more than 5 parts by mass, the hue is liable to deteriorate. Further, too much content of the compound (B) is not preferable from an economic viewpoint. From the viewpoint of improving impact resistance and fluidity in a good balance, the content of the compound (B) is preferably 0.03 to 4 parts by mass, more preferably 0.04 to 3 parts by mass, further preferably 0.05 to 2 parts by mass, and still more preferably 0.1 to 0.3 parts by mass with respect to (A) 100 parts by mass of the polyolefin resin. The compound (B) may react with the polyolefin resin (A), and the like, under heating conditions in melt-kneading and the like in the production of the polyolefin resin composition of the present invention. Thus, the content of the compound (B) in the polyolefin resin composition of the present invention includes both the amount of the unreacted compound (B) that is present as it is in the polyolefin resin composition, and the amount of the compound (B) having another structure through a reaction with the polyolefin resin and the like. Thus, the content of the compound (B) in the polyolefin resin composition of the present invention may be considered as the amount of the compound (B) added in the production of the polyolefin resin composition (charged amount).

The content of the unreacted compound (B) that is present as it is in the polyolefin resin composition varies depending on the production conditions of the polyolefin resin composition, and is preferably 1 to 100%, more preferably 1 to 95%, further preferably 1 to 90%, still more preferably about 2 to 90% with respect to the amount of the compound (B) added in the production of the polyolefin resin composition (charged amount). Thus, the content of the unreacted compound (B) in the polyolefin resin composition is preferably 0.0001 to 5 parts by mass, more preferably 0.0001 to 4.95 parts by mass, further preferably 0.0001 to 4.5 parts by mass, and still more preferably 0.0002 to 4 parts by mass with respect to (A) 100 parts by mass of the polyolefin resin. The content of the unreacted compound (B) in the polyolefin resin composition may be measured, for example, by subjecting a polyolefin resin pellet to Soxhlet extraction with a solvent such as chloroform, and then carrying out liquid chromatography using the solvent as the measurement sample.

In the polyolefin resin composition of the present invention, the content of (C) the organic peroxide is 0.01 to 5 parts by mass with respect to (A) 100 parts by mass of the polyolefin resin. When the content of the organic peroxide is less than 0.01 parts by mass, the fluidity and impact resistance of the polyolefin resin composition cannot be sufficiently increased. In contrast, when the content of the organic peroxide is more than 5 parts by mass, the impact resistance of the molded body to be obtained is reduced. From the viewpoint of easily achieving both fluidity and impact resistance, the content of the organic peroxide is preferably 0.03 to 3 parts by mass, more preferably 0.04 to 2 parts by mass, further preferably 0.05 to 1 part by mass, and still more preferably 0.05 to 0.5 with respect to (A) 100 parts by mass of the polyolefin resin. Also, the organic peroxide (C) may react with the polyolefin resin (A) and the like, under heating conditions in melt-kneading and the like in the production of the polyolefin resin composition of the present invention. Thus, the content of the organic peroxide (C) in the polyolefin resin composition of the present invention includes both the amount of the unreacted organic peroxide (C) that is present as it is in the polyolefin resin composition, and the amount of the organic peroxide (C) having another structure through a reaction with the polyolefin resin and the like. Thus, the content of the organic peroxide (C) in the polyolefin resin composition of the present invention may be considered as the amount of the organic peroxide (C) added in the production of the polyolefin resin composition (charged amount).

The content of the unreacted organic peroxide (C) that is present as it is in the polyolefin resin composition varies depending on the production conditions of the polyolefin resin composition, and is preferably 0.1 to 10%, more preferably 0.1 to 5%, and further preferably about 1 to 3% with respect to the amount of the organic peroxide (C) added in the production of the polyolefin resin composition (charged amount). Thus, the content of the unreacted organic peroxide (C) in the polyolefin resin composition is preferably 0.00001 to 0.5 parts by mass, more preferably 0.00001 to 0.4 parts by mass, further preferably 0.00001 to 0.3 parts by mass, still more preferably 0.00001 to 0.25 parts by mass, and especially preferably 0.00001 to 0.15 parts by mass with respect to (A) 100 parts by mass of the polyolefin resin. The content of the unreacted organic peroxide (C) in the polyolefin resin composition may be measured, for example, by subjecting a polyolefin resin pellet to Soxhlet extraction with a solvent such as chloroform, and then carrying out liquid chromatography using the solvent as the measurement sample.

The content of the polyolefin resin contained in the polyolefin resin composition of the present invention is preferably 60 to 99.9% by mass, more preferably 70 to 99.9% by mass, and further preferably 75 to 99.0% by mass, based on the total amount of the polyolefin resin composition.

In the polyolefin resin composition of the present invention, the mole ratio of the compound (B):the organic peroxide (C) is preferably 1:10 to 10:1, more preferably 5:1 to 1:1, and further preferably 3:1 to 1.2:1, from the viewpoint of improving impact resistance. When the mole ratio of the compound (B) to the organic peroxide is within the above range, yellowness is easily lowered and both the fluidity and impact resistance of the polyolefin resin composition are easily achieved. From a similar viewpoint, the amount by mole of the compound (B) contained in the polyolefin resin composition is preferably higher than the amount by mole of the organic peroxide (C). In this case, the mole ratio of the compound (B):the organic peroxide (C) is preferably 10:1 to 1.01:1, more preferably 5:1 to 1.1:1, and still more preferably 3:1 to 1.2:1.

### <Compound (B)>

The polyolefin resin composition of the present invention contains 0.01 to 5 parts by mass of (B) the compound having at least one (meth)acrylate group, and one hydrogen bonding group or one (meth)acrylate group with respect to 100 parts by mass of the polyolefin resin. The compound (B) is not particularly limited as long as it has the above functional groups, and the polyolefin resin composition of the present invention may contain one compound (B), or may contain two or more compounds (B). In the present specification, the (meth)acrylate group refers to an acrylate group or a methacrylate group. In the present specification, the hydrogen bonding group is not particularly limited, as long as it is a hydrogen bondable group, and examples thereof include a hydroxyl group, an amino group (primary and secondary amino groups), a thiol group, a carboxyl group, a sulfonic acid group, a phosphoric acid group, an amide group, and a carbamate group. When the compound (B) has a hydrogen bonding group, the hydrogen bonding group is preferably selected from the group consisting of a hydroxyl group, an amino group, a thiol group, a carboxyl group, a sulfonic acid group, and a phosphoric acid group, more preferably selected from the group consisting of a hydroxyl group and an amino group, and further preferably a hydroxyl group, from the viewpoint of easily lowering yellowness and easily achieving both fluidity and impact resistance.

When the compound (B) has a hydrogen bonding group, the number of the hydrogen bonding group in the compound (B) may be at least one, and from the viewpoint of easily reducing the color tone (yellowness) derived from the polyolefin resin composition itself and easily improving impact resistance and fluidity, it is preferably one to three, more preferably one or two, and further preferably one. When the compound (B) has no hydrogen bonding group and has two or more (meth)acrylate groups, the number of (meth)acrylate group in the compound (B) may be at least two, and from the viewpoint of easily reducing the color tone (yellowness) derived from the polyolefin resin composition itself and easily improving impact resistance and fluidity, it is preferably two to three, and more preferably two. Therefore, from a similar viewpoint, the compound (B) preferably has one (meth)acrylate group and one hydrogen bonding group, or two (meth)acrylate groups.

In a preferred aspect of the present invention, (B) the compound having at least one (meth)acrylate group, and one hydrogen bonding group or one (meth)acrylate group is represented by formula (I):
[Formula 9] 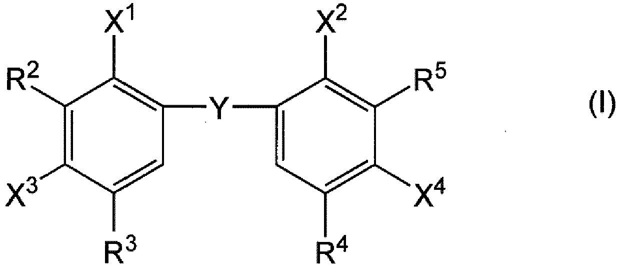 wherein
X¹ and X² each independently represent a hydrogen bonding group or a group represented by -OC(=O)CR¹=CH₂ and X³ and X⁴ each independently represent a hydrogen atom or a linear or branched alkyl group having 1 to 9 carbon atoms, or X¹ and X² each independently represent a hydrogen atom or a linear or branched alkyl group having 1 to 9 carbon atoms and X³ and X⁴ each independently represent a hydrogen bonding group or a group represented by -OC(=O)CR¹=CH₂, provided that at least one of X¹ to X⁴ represents a group represented by -OC(=O)CR¹=CH₂, and R¹ represents a hydrogen atom or a methyl group;
Y represents a single bond, a sulfide bond, or a linear or branched alkylene group having 1 to 9 carbon atoms; and
R² and R⁵ each independently represent a hydrogen atom or a linear or branched alkyl group having 1 to 9 carbon atoms, and R³ and R⁴ each independently represent a linear or branched alkyl group having 1 to 9 carbon atoms. The polyolefin resin composition preferably contains the compound (B) represented by above formula (I), because the color tone derived from the polyolefin resin composition itself is easily reduced and the impact resistance and fluidity of the resin composition are easily improved. In this aspect, the polyolefin resin composition of the present invention may contain one compound represented by formula (I), or may contain two or more compounds represented by formula (I).

In formula (I), X¹, X², X³, and X⁴ may represent a hydrogen bonding group, -OC(=O)CR¹=CH₂ [R¹ represents a hydrogen atom or a methyl group], a hydrogen atom, or a linear or branched alkyl group having 1 to 9 carbon atoms, under the above conditions. Examples of the hydrogen bonding group are as described above, and it is preferably selected from the group consisting of a hydroxyl group, an amino group, a thiol group, a carboxyl group, a sulfonic acid group, and a phosphoric acid group, more preferably selected from the group consisting of a hydroxyl group and an amino group, and further preferably a hydroxyl group. The group represented by - OC(=O)CR¹=CH₂ is an acryloyloxy group or a methacryloyloxy group. Examples of the linear or branched alkyl group having 1 to 9 carbon atoms include the groups described below with respect to R^{2a} to R^{5a} in formula (Ia).

In formula (I), R¹ represents a hydrogen atom or a methyl group; R² and R⁵ each independently represent a hydrogen atom or a linear or branched alkyl group having 1 to 9 carbon atoms; and R³ and R⁴ each independently represent a linear or branched alkyl group having 1 to 9 carbon atoms. Examples of the linear or branched alkyl group having 1 to 9 carbon atoms include the groups described below with respect to R^{2a} to R^{5a} in formula (Ia).

In formula (I), Y represents a single bond, a sulfide bond, or a linear or branched alkylene group having 1 to 9 carbon atoms. Examples of the linear or branched alkylene group having 1 to 9 carbon atoms include the groups described below with respect to Y^{a} in formula (Ia).

In a preferred aspect of the present invention, the compound (B) represented by formula (I) is preferably a compound wherein X¹ represents a hydrogen bonding group or a group represented by -OC(=O)CR¹=CH₂; X² represents a group represented by -OC(=O)CR¹=CH₂; X³ and X⁴ represent a hydrogen atom; Y represents a single bond or a linear or branched alkylene group having 1 to 9 carbon atoms; R¹ represents a hydrogen atom or a methyl group; and R² to R⁵ each independently represent a linear or branched alkyl group having 1 to 9 carbon atoms. In this case, the compound (B) is a compound represented by formula (I-1) :
[Formula 10] 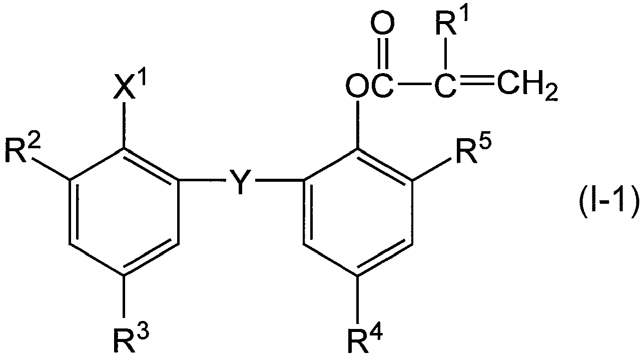 wherein X¹ represents a hydrogen bonding group or a group represented by -OC(=O)CR¹=CH₂; Y represents a single bond or a linear or branched alkylene group having 1 to 9 carbon atoms; R¹ represents a hydrogen atom or a methyl group; and R² to R⁵ each independently represent a linear or branched alkyl group having 1 to 9 carbon atoms.

In the above formula (I-1), X¹ represents a hydrogen bonding group or a group represented by - OC(=O)CR¹=CH₂, and from the viewpoint of easily achieving both fluidity and impact resistance, X¹ preferably represents a hydrogen bonding group, more preferably a hydroxyl group, an amino group, a thiol group, a carboxyl group, a sulfonic acid group, or a phosphoric acid group, further preferably a hydroxyl group or an amino group, and still more preferably a hydroxyl group.

In the above formula (I-1), from the viewpoint of easily achieving both the fluidity and impact resistance of the polyolefin resin composition, Y preferably represents a methylene group which optionally has an alkyl group having 1 to 8 carbon atoms as a side chain, further preferably a methylene group which optionally has an alkyl group having 1 to 3 carbon atoms as a side chain, and still more preferably a methylmethylene group or a methylene group.

In the above formula (I-1), R² to R⁵ each independently represent a linear or branched alkyl group having 1 to 9 carbon atoms, and from the viewpoint of easily achieving both the fluidity and impact resistance of the polyolefin resin composition, preferably each independently represent a group selected from the group consisting of a methyl group, a tert-butyl group, and a 1,1-dimethylpropyl group.

In a preferred aspect of the present invention, the compound (B) is a compound having one (meth)acrylate group and at least one hydrogen bonding group, and more preferably a compound represented by formula (Ia):
[Formula 11] 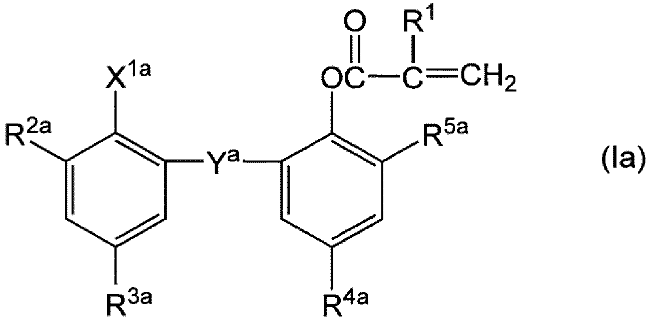 wherein
X^{1a} represents a hydrogen bonding group;
Y^{a} represents a single bond or a linear or branched alkylene group having 1 to 9 carbon atoms;
R¹ represents a hydrogen atom or a methyl group, and
R^{2a} to R^{5a} each independently represent a linear or branched alkyl group having 1 to 9 carbon atoms. In this aspect, the polyolefin resin composition of the present invention may contain one compound represented by formula (Ia), or may contain two or more compounds represented by formula (Ia).

X^{1a} in formula (Ia)represents a hydrogen bonding group and examples of the hydrogen bonding group include the groups described above. From the viewpoint of easily achieving both fluidity and impact resistance, X^{1a} is preferably a hydroxyl group, an amino group, a thiol group, a carboxyl group, a sulfonic acid group or a phosphoric acid group, more preferably a hydroxyl group or an amino group, and further preferably a hydroxyl group.

Y^{a} in formula (Ia)represents a single bond or a linear or branched alkylene group having 1 to 9 carbon atoms. From the viewpoint of easily lowering yellowness and easily achieving both fluidity and impact resistance, the number of carbon atoms of the linear or branched alkylene group having 1 to 9 carbon atoms is preferably 1 to 6, more preferably 1 to 4, and particularly preferably 1 to 3. Examples of the linear or branched alkylene group having 1 to 9 carbon atoms include a methylene group, an ethylene group, a n-propylene group, an isopropylene group, a n-butylene group, a sec-butylene group, a n-pentylene group, a 2-methyl-butylene group, a 3-methylbutylene group, a 2-ethyl-propylene group, n-hexylene group, a n-heptylene group, a n-octylene group, and a n-nonylene group. From the viewpoint of easily achieving both the fluidity and impact resistance of the polyolefin resin composition, Y^{a} in formula (Ia) preferably represents a methylene group which optionally has an alkyl group having 1 to 8 carbon atoms, further preferably a methylene group which optionally has an alkyl group having 1 to 3 carbon atoms, and particularly preferably a methylmethylene group or a methylene group.

R¹ in formula (Ia) represents a hydrogen atom or a methyl group, and from the viewpoint of improving impact resistance, R¹ in formula (Ia) represents a hydrogen atom.

R^{2a} to R^{5a} in formula (Ia) each independently represent a linear or branched alkyl group having 1 to 9 carbon atoms. From the viewpoint of easily achieving both the fluidity and impact resistance of the polyolefin resin composition, the number of carbon atoms of the linear or branched alkyl group having 1 to 9 carbon atoms is preferably 1 to 6, and more preferably 1 to 5. Examples of the linear or branched alkyl group having 1 to 9 carbon atoms include a methyl group, an ethyl group, a n-propyl group, an isopropyl group, a n-butyl group, a sec-butyl group, a tert-butyl group, a n-pentyl group, a 1,1-dimethylpropyl group, a 2-methyl-butyl group, a 3-methylbutyl group, a 2-ethyl-propyl group, a n-hexyl group, a n-heptyl group, a n-octyl group, a tert-octyl group, and a n-nonyl group. From the viewpoint of easily achieving both the fluidity and impact resistance of the polyolefin resin composition, R^{2a} to R^{5a} in formula (Ia) are preferably each independently a group selected from the group consisting of a methyl group, a tert-butyl group, and a 1,1-dimethylpropyl group.

In a preferred aspect of the present invention, formula (Ia) is represented by formula (Ia'):
[Formula 12] 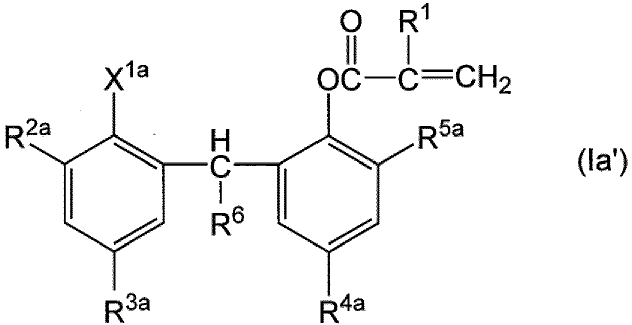 wherein X^{1a}, R¹ and R^{2a} to R^{5a} are as defined for X^{1a}, R¹ and R^{2a} to R^{5a} in formula (Ia), respectively; and R⁶ represents a hydrogen atom or a methyl group. In this aspect, the polyolefin resin composition of the present invention may contain one compound represented by formula (Ia'), or may contain two or more compounds represented by formula (Ia').

X^{1a}, R¹ and R^{2a} to R^{5a} in formula (Ia') are as defined for X^{1a}, R¹ and R^{2a} to R^{5a} in formula (Ia), respectively, and the above description about X^{1a}, R¹ and R^{2a} to R^{5a} in formula (Ia) also similarly applies to X^{1a}, R¹ and R^{2a} to R^{5a} in formula (Ia').

For example, X^{1a} in formula (Ia') is preferably a hydroxyl group or an amino group, and more preferably a hydroxyl group. R^{2a} to R^{5a} in formula (Ia') is preferably each independently a group selected from the group consisting of a methyl group, a tert-butyl group, and a 1,1-dimethylpropyl group.

In a preferred aspect of the present invention, the compound represented by formula (Ia') is a compound wherein X^{1a} represents a hydroxyl group; R¹ represents a hydrogen atom; R^{2a} to R^{5a} represents a 1,1-dimethylpropyl group; and R⁶ represents a methyl group, from the viewpoint of easily reducing the color tone derived from the polyolefin resin composition itself and easily improving the fluidity and impact resistance of the polyolefin resin composition. Such a compound is, for example, a compound commercially available as Sumilizer GS from SUMITOMO CHEMICAL COMPANY, LIMITED.

In another preferred aspect of the present invention, the compound represented by formula (Ia') is a compound wherein X^{1a} represents a hydroxyl group; R¹ and R⁶ represent a hydrogen atom; R^{2a} and R^{5a} represent a t-butyl group; R^{3a} and R^{4a} represent a methyl group, from the viewpoint of easily reducing the color tone derived from the polyolefin resin composition itself and easily improving the fluidity and impact resistance of the polyolefin resin composition. Such a compound is, for example, a compound commercially available as Sumilizer GM from SUMITOMO CHEMICAL COMPANY, LIMITED.

In another preferred aspect of the present invention, the compound represented by formula (I-1) is a compound wherein X^{1a} is a hydrogen bonding group or a group represented by -OC(=O)CR¹=CH₂, preferably a hydrogen bonding group, and more preferably a hydroxyl group; R¹ represents a hydrogen atom; R^{2a} to R^{5a} represent a methyl group; and Y is a methylmethylene group, from the viewpoint of easily reducing the color tone derived from the polyolefin resin composition itself and easily improving the fluidity and impact resistance of the polyolefin resin composition. Such a compound is a compound represented by the following formula (1c): 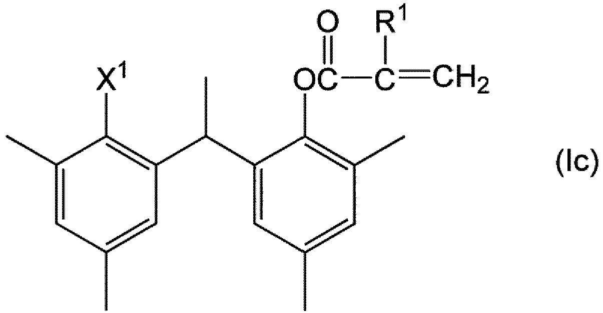 wherein X¹ represents a hydrogen bonding group or a group represented by -OC(=O)CR¹=CH₂; and R¹ represents a hydrogen atom.

The above compound represented by formula (Ic) is a compound that can prevent the change in color of a resin and can improve the impact resistance and fluidity of a resin composition when used as an additive for a resin such as a polyolefin resin. Therefore, the compound is an effective compound as an additive for a resin, preferably an additive for a polyolefin resin, and more preferably an anti-tarnish agent, an impact resistance improving agent, a fluidity improving agent, and the like for the resin. The present invention also provides the above compound represented by formula (Ic). The compound represented by formula (Ic) can be produced, for example, by the method described in Examples.

In another preferred aspect of the present invention, the compound (B) represented by formula (I) is preferably a compound wherein X¹ and X² represent a linear or branched alkyl group having 1 to 9 carbon atoms; X³ represents a hydrogen bonding group or a group represented by -OC(=O)CR¹=CH₂; X⁴ represents a group represented by -OC(=O)CR¹=CH₂; Y represents a sulfide bond; R¹ represents a hydrogen atom or a methyl group; R² and R⁵ represent a hydrogen atom; and R³ and R⁴ each independently represent a linear or branched alkyl group having 1 to 9 carbon atoms. In this case, the compound (B) is a compound represented by formula (1-2):
[Formula 13] 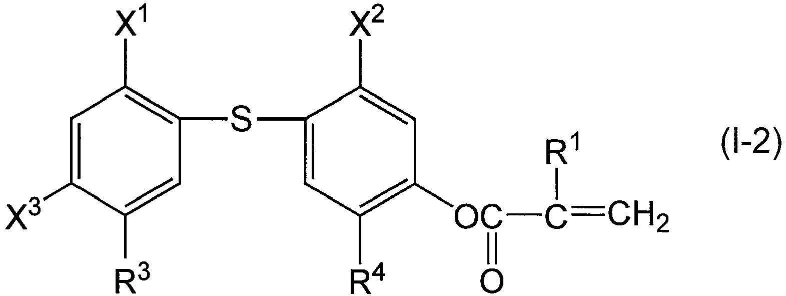 wherein X¹ and X² each independently represent a linear or branched alkyl group having 1 to 9 carbon atoms; X³ represents a hydrogen bonding group or a group represented by -OC(=O)CR¹=CH₂; R¹ represents a hydrogen atom or a methyl group; and R³ and R⁴ each independently represent a linear or branched alkyl group having 1 to 9 carbon atoms.

In the above formula (1-2), X³ represents a hydrogen bonding group or a group represented by - OC(=O)CR¹=CH₂, and from the viewpoint of easily achieving both fluidity and impact resistance, X³ preferably represents a hydrogen bonding group, more preferably a hydroxyl group, an amino group, a thiol group, a carboxyl group, a sulfonic acid group, or a phosphoric acid group, further preferably a hydroxyl group or an amino group, and still more preferably a hydroxyl group.

In the above formula (1-2), X¹ and X² each independently represent a linear or branched alkyl group having 1 to 9 carbon atoms, and from the viewpoint of easily achieving both the fluidity and impact resistance of the polyolefin resin composition, X¹ and X² preferably each independently represent a group selected from the group consisting of a methyl group, a tert-butyl group, and a 1,1-dimethylpropyl group, and more preferably a methyl group.

In the above formula (1-2), R³ and R⁴ each independently represent a linear or branched alkyl group having 1 to 9 carbon atoms, and from the viewpoint of easily achieving both the fluidity and impact resistance of the polyolefin resin composition, R³ and R⁴ preferably each independently represent a group selected from the group consisting of a methyl group, a tert-butyl group, and a 1,1-dimethylpropyl group, and more preferably a tert-butyl group.

In another preferred aspect of the present invention, the compound represented by formula (1-2) is a compound wherein X¹ and X² represent a methyl group; X³ represents a hydrogen bonding group or a group represented by -OC(=O)CR¹=CH₂; R¹ represents a hydrogen atom; and R³ and R⁴ represent a methyl group, from the viewpoint of easily reducing the color tone derived from the polyolefin resin composition itself and easily improving the fluidity and impact resistance of the polyolefin resin composition. Such a compound is a compound represented by the following formula (Id):
[Formula 14] 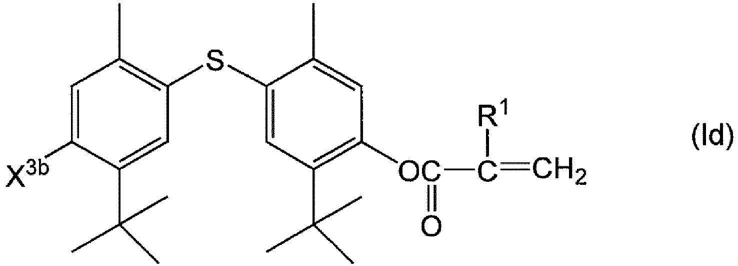 wherein X^{3b} represents a hydrogen bonding group or a group represented by -OC(=O)CR¹=CH₂; and R¹ represents a hydrogen atom.

The compound represented by the above formula (Id) is a compound that can prevent the change in color of a resin and can improve the impact resistance and fluidity of a resin composition when used as an additive for a resin such as a polyolefin resin. Therefore, the compound is an effective compound as an additive for resin, preferably an additive for polyolefin resin, and more preferably an anti-tarnish agent, an impact resistance improving agent, a fluidity improving agent, and the like for the resin. The present invention also provides a compound represented by the above formula (Id). The compound represented by formula (Id) can be produced, for example, by the method described in Examples.

### <(C) Organic Peroxide>

The polyolefin resin composition of the present invention contains 0.01 to 5 parts by mass of (C) the organic peroxide with respect to 100 parts by mass of the polyolefin resin. In the present invention, the organic peroxide is considered to have the functions of decomposing the polyolefin resin by, for example, cleaving polyolefin resin chains under high temperature conditions, and improving the fluidity of the polyolefin resin. It is also considered that any reaction caused between the organic peroxide and the compound (B) results in a function of improving the function of the compound (B) of finely dispersing the polyolefin resin. Examples of the organic peroxide include an alkyl peroxide compound, a diacyl peroxide compound, a peroxy ester compound, and a peroxycarbonate compound.

Examples of the alkyl peroxide compound include dicumyl peroxide, di-tert-butyl peroxide, di-tert-butyl cumyl peroxide, 2,5-dimethyl-2,5-di(tert-butylperoxy)hexane, 2,5-dimethyl-2,5-di(tert-butylperoxy)hexine-3, tert-butyl cumyl, 1,3-bis(tert-butylperoxyisopropyl)benzene, 1,4-bis(tert-butylperoxyisopropyl)benzene, and 3,6,9-triethyl-3,6,9-trimethyl-1,4,7-triperoxonane.

Examples of the diacyl peroxide compound include benzoyl peroxide, lauroyl peroxide, and decanoyl peroxide.

Examples of the peroxy ester compound include 1,1,3,3-tetramethylbutyl peroxy neodecanoate, α-cumyl peroxy neodecanoate, tert-butyl peroxy neodecanoate, tert-butyl peroxy neoheptanoate, tert-butyl peroxy pivalate, tert-hexyl peroxy pivalate, 1,1,3,3-tetramethylbutyl peroxy-2-ethylhexanoate, tert-amyl peroxyl-2-ethylhexanoate, tert-butyl peroxy-2-ethylhexanoate, tert-butyl peroxyisobutyrate, di-tert-butyl peroxyhexahydroterephthalate, tert-amyl peroxy 3,5,5-trimethylhexanoate, tert-butyl peroxy 3,5,5-trimethylhexanoate, tert-butyl peroxy acetate, tert-butyl peroxy benzoate, and di-butyl peroxy trimethyl adipate.

Examples of the peroxycarbonate compound include di-3-methoxybutyl peroxydicarbonate, di(2-ethylhexyl) peroxydicarbonate, diisopropyl peroxycarbonate, tert-butylperoxy isopropylcarbonate, di(4-tert-butylcyclohexyl) peroxydicarbonate, dicetylperoxydicarbonate, and dimyristylperoxydicarbonate.

From the viewpoint of easily reducing the color tone derived from the polyolefin resin composition itself and easily improving the impact resistance and fluidity of the polyolefin resin composition, the organic peroxide is preferably an alkyl peroxide compound, and more preferably at least one selected from the group consisting of 2,5-dimethyl-2,5-di(tert-butylperoxy)hexane, 1,3-bis(tert-butylperoxyisopropyl)benzene, and 1,4-bis(tert-butylperoxyisopropyl)benzene.

### <(A) Polyolefin Resin>

Examples of the polyolefin resin contained in the polyolefin resin composition of the present invention include an ethylene homopolymer, an α-olefin homopolymer having 3 or more carbon atoms, copolymers of two or more monomers selected from the group consisting of ethylene and α-olefin having 3 or more carbon atoms, and copolymers of these and a monomer other than ethylene or α-olefin. The polyolefin resin composition of the present invention may contain one polyolefin resin or two or more polyolefin resins as the polyolefin resin.

The polyolefin resin contained in the polyolefin resin composition of the present invention preferably includes a propylene-based resin. In the present specification, the propylene-based resin is a resin selected from the group consisting of a propylene homopolymer and a propylene-based copolymer. The propylene homopolymer is a homopolymer of propylene, and the propylene-based copolymer is a copolymer of propylene and ethylene and/or an α-olefin having 4 or more carbon atoms. The polyolefin resin contained in the polyolefin resin composition may contain one propylene-based resin, or two or more propylene-based resins.

The propylene-based resin is a homopolymer of propylene, and/or a copolymer of propylene and ethylene and/or an α-olefin having 4 or more carbon atoms. Examples of the α-olefin having 4 or more carbon atoms preferably include α-olefins having 4 to 10 carbon atoms, and more preferably α-olefins having 4 to 8 carbon atoms. Examples of the α-olefins having 4 or more carbon atoms preferably include 1-butene, 1-pentene, 1-hexene, 4-methyl-1-pentene, 1-octene, and 1-decene, and more preferably include 1-butene, 1-hexene, and 1-octene. The propylene-based copolymer may preferably be a copolymer of one or two or more of these α-olefins and propylene. However, when the propylene-based resin includes a monomer unit derived from ethylene, the amount of the monomer unit derived from ethylene is less than 20 mass, based on the total amount of the propylene-based resin.

When the polyolefin resin contains the propylene-based resin, the intrinsic viscosity of the propylene-based resin is preferably 0.1 to 5 dl/g, more preferably 0.3 to 4 dl/g, and further preferably 0.5 to 3 dl/g, from the viewpoint of the rigidity of the molded body. The measurement method of the intrinsic viscosity is as described in Examples.

In a preferred aspect of the present invention, the polyolefin resin contained in the polyolefin resin composition further contains an ethylene-based copolymer, in addition to the propylene-based resin. The ethylene-based copolymer is a copolymer of ethylene and an α-olefin having 3 or more carbon atoms. Examples of the α-olefin having 3 or more carbon atoms preferably include α-olefins having 3 to 10 carbon atoms, more preferably include α-olefins having 3 to 8 carbon atoms. Examples of the α-olefins having 3 or more carbon atoms preferably include propylene, 1-butene, 1-pentene, 1-hexene, 4-methyl-1-pentene, 1-octene, and 1-decene, and more preferably include propylene, 1-butene, 1-hexene, and 1-octene. The ethylene-based copolymer may preferably be a copolymer of ethylene and one or two or more of these α-olefins. However, when the ethylene-based copolymer includes a monomer unit derived from propylene, the amount of the monomer unit derived from ethylene in the copolymer is 20% by mass or more, based on the total amount of the ethylene-based copolymer.

The content of the monomer unit derived from ethylene in the ethylene-based copolymer is preferably 20% by mass or more, more preferably 20 to 99.5% by mass, still more preferably 25 to 99% by mass, and particularly preferably 28 to 90% by mass, based on the total amount of the ethylene-based copolymer, from the viewpoint of the impact resistance of the molded body.

The content of the monomer unit derived from the α-olefin having 3 or more carbon atoms in the ethylene-based copolymer is preferably 80% by mass or less, more preferably 0.5 to 80% by mass, still more preferably 1 to 75% by mass, and particularly preferably 10 to 72% by mass, based on the total amount of the monomer unit derived from ethylene and the monomer unit derived from the α-olefin having 3 or more carbon atoms contained in the copolymer, from the viewpoint of economy.

In a preferred aspect of the present invention in which the polyolefin resin contained in the polyolefin resin composition further contains the ethylene-based copolymer in addition to the propylene-based resin, the content of the propylene-based resin is preferably 50% by mass or more, more preferably 55% by mass or more, and further preferably 60% by mass or more, based on the total amount of the propylene-based resin and ethylene-based copolymer contained in the polyolefin resin composition, from the viewpoint of easily achieving impact resistance and rigidity in a good balance. The content of the ethylene-based copolymer is preferably 50% by mass or less, more preferably 45% by mass or less, and further preferably 40% by mass or less, based on the total amount of the propylene-based resin and ethylene-based copolymer contained in the polyolefin resin composition, from the viewpoint of easily achieving impact resistance and rigidity in a good balance. When the polyolefin resin contained in the polyolefin resin composition contains two or more ethylene-based copolymers, the total amount of the ethylene-based copolymers is only required to be within the above range.

In a preferred aspect of the present invention in which the polyolefin resin contained in the polyolefin resin composition contains at least a propylene-based resin and an ethylene-based copolymer, the polyolefin resin may contain a propylene-based resin and an ethylene-based copolymer which are separately produced, or may contain a resin, also referred to as an impact copolymer, which is produced by polymerizing a homopolymer of propylene using a polymerization catalyst, and subsequently and continuously polymerizing an ethylene-propylene copolymer, for example, allowing an ethylene gas to coexist. The impact copolymer is a resin also referred to as the ethylene-propylene block copolymer. Examples of such an impact copolymer include the polymer disclosed in Japanese Patent Laid-Open No. 2004-182981. The polyolefin resin may be produced by further adding an ethylene-based copolymer to the above resin also referred to as the impact copolymer. When resins separately produced are mixed, mixing may be carried out by melt-kneading.

### <Other Components>

### (Filler)

The polyolefin resin composition may contain an inorganic filler to improve the mechanical properties of the molded body to be obtained. Examples of the inorganic filler include calcium carbonate, calcium hydroxide, calcium oxide, barium sulfate, mica, crystalline calcium silicate, talc, and fibrous magnesium oxysulfate. The inorganic filler is preferably talc or fibrous magnesium oxysulfate, and more preferably talc. These inorganic fillers may be used alone or in combination of two or more. When the inorganic filler is used, the content thereof is preferably 1 to 25 parts by mass with respect to 100 parts by mass of the polyolefin resin, from the viewpoint of easily increasing the rigidity and impact strength of the molded body to be obtained.

### (Additive)

Examples of additives that may be added to the polyolefin resin composition include neutralizing agents, antioxidants, processing stabilizers, ultraviolet absorbers, hindered amine light stabilizers, nucleating agents, clarifying nucleating agents, lubricants, processing aids, metal soaps, colorants (pigments such as carbon black and titanium oxide), foaming agents, antimicrobial agents, plasticizers, flame retardants, cross-linking agents, cross-linking aids, and brightening agents. These additives may be used alone or in combination of two or more. When the above additives are used, the content thereof is preferably 1 to 25 parts by mass with respect to 100 parts by mass of the polyolefin resin, from the viewpoint of easily increasing the rigidity and impact strength of the molded body to be obtained.

YI of the polyolefin resin composition of the present invention is preferably 20 or less, more preferably 15 or less, further preferably 12 or less, still more preferably 10 or less, particularly preferably 8 or less, and most preferably 6 or less, from the viewpoint of easily improving the quality of the molded body to be obtained from the resin composition of the present invention. For example, YI of the polyolefin resin composition may be measured with a colorimeter using a sheet obtained by subjecting the resin composition to press molding as the measurement sample, and for example, measured by the method described in Examples.

### <Method for Producing Polyolefin Resin Composition>

The polyolefin resin composition of the present invention can be produced by a production method including at least a step of mixing (A) a polyolefin resin, (B) a compound having at least one (meth)acrylate group, and one hydrogen bonding group or one (meth)acrylate group, and (C) an organic peroxide together by melt-kneading a mixture obtained by dry-mixing in advance. The present invention also provides a method for producing the polyolefin resin composition. Although it is not clear in what state the polyolefin resin is finely dispersed, the fluidity and impact resistance of the polyolefin resin can be improved according to the production method of the present invention including at least a step of mixing (A) the polyolefin resin, the compound (B), and (C) the organic peroxide together by melt-kneading a mixture obtained by dry-mixing in advance. In the production method of the present invention, melt-kneading of the mixture obtained by dry-mixing (A) the polyolefin resin, the compound (B), and (C) the organic peroxide in advance may be carried out by dry-mixing all of (A) the polyolefin resin, the compound (B), and the organic peroxide (C) and then melt-kneading, or may be carried out by dry-mixing at least a part of (A) the polyolefin resin, and the compound (B) and/or the organic peroxide (C) and melt-kneading, and then dry-mixing the obtained mixture and the remaining part again and melt-kneading.

The polyolefin resin composition of the present invention may be produced by using a masterbatch in which the compound (B) is mixed with a resin in advance and/or a masterbatch in which (C) the organic peroxide is mixed with a resin in advance, and mixing the masterbatch with (A) the polyolefin resin. When these masterbatches are used, these masterbatches may be side-fed and mixed while melt-kneading (A) the polyolefin resin with an extruder to give the polyolefin resin composition of the present invention.

Therefore, the present invention also provides a masterbatch containing the compound (B) and a resin, and preferably a masterbatch at least containing the compound (B) and at least one resin selected from the group consisting of a polyolefin-based resin and an ethylene-based copolymer. The masterbatch of the present invention contains the compound (B) in an amount of preferably 10 to 50 parts by mass, more preferably 20 to 48 parts by mass, and further preferably 25 to 46 parts by mass with respect to 100 parts by mass of the resin (preferably at least one resin selected from the group consisting of a polyolefin-based resin and an ethylene-based copolymer, and more preferably an ethylene-based copolymer). Examples of the polyolefin-based resin and the ethylene-based copolymer contained in the masterbatch include the resins described above as the polyolefin-based resin and the ethylene-based copolymer contained in the polyolefin resin composition of the present invention. The masterbatch of the present invention may contain one resin or two or more resins in combination.

The masterbatch of the present invention may further contain an organic peroxide. In this case, the amount of the organic peroxide is preferably 5 to 25 parts by mass, more preferably 5 to 20 parts by mass, and further preferably 5 to 15 parts by mass with respect to 100 parts by mass of the resin (preferably at least one resin selected from the group consisting of a polyolefin-based resin and an ethylene-based copolymer, and more preferably an ethylene-based copolymer).

The method for producing the masterbatch of the present invention is not particularly limited, but from the viewpoint of easily suppressing the decomposition of the compound (B) by heating and the like, it is preferable to use a method of kneading the compound (B) and the resin at a temperature lower than the melting point of the compound (B). From such a viewpoint, the masterbatch is preferably produced by kneading the compound (B) and the resin, optionally with an organic peroxide and other additives at a temperature of preferably 150°C or less, more preferably 120°C or less, and further preferably 100°C or less. From the viewpoint of easily suppressing the decomposition of the compound (B) and easily producing a masterbatch in which the compound (B) is uniformly dispersed, the resin to be contained in the masterbatch is preferably at least one resin selected from the group consisting of a polyolefin-based resin and an ethylene-based copolymer, and more preferably an ethylene-based copolymer.

Specific examples of the method for producing the polyolefin resin composition of the present invention include:
production method I: a production method including step (1) of dry-mixing (A) the polyolefin resin, the compound (B), and, (C) the organic peroxide to give a mixture; and step (2) of melt-kneading the mixture obtained in step (1) to give the polyolefin resin composition of the present invention; or
production method II: a production method including step (1a) of dry-mixing (A) the polyolefin resin and a part of (C) the organic peroxide to give a first dry mixture; step (2a) of melt-kneading the dry mixture obtained in step (1a) to give a first premixture; step (1b) of dry-mixing the first premixture obtained in step (2a), the compound (B), and another part of (C) the organic peroxide to give a second dry mixture; and step (2b) of melt-kneading the dry mixture obtained in step (1b) to give the polyolefin resin composition of the present invention.

When the polyolefin resin contained in the polyolefin resin composition contains at least a propylene-based resin and an ethylene-based copolymer (particularly when the content of the ethylene-based resin is preferably 50% by mass or more, and more preferably 60% by mass or more, based on the total amount of the propylene-based resin and ethylene-based copolymer contained in the polyolefin resin composition), it is preferable to melt-knead (A) the polyolefin resin and (C) the organic peroxide before (A) the polyolefin resin and the compound (B) are melt-kneaded, from the viewpoint of easily improving the impact resistance and fluidity of the polyolefin resin composition. From the viewpoint of easily improving the impact resistance and fluidity of the polyolefin resin composition, it is more preferable to melt-knead (A) the polyolefin resin and a part of (C) the organic peroxide, and then to add the compound (B) and another part of (C) the organic peroxide (remaining part) to the obtained mixture. Thus, in the aspect, the above production method (II) is preferably used. It is considered that, due to the addition of the organic peroxide in at least two portions, the organic peroxide not only efficiently decomposes the polyolefin resin and easily improves the fluidity of the resin, but also the organic peroxide contributes to the reaction and/or interaction between the compound (B) and the polyolefin resin, whereby the fine dispersibility of the polyolefin resin is improved and the impact resistance of the polyolefin resin composition is easily improved.

When the polyolefin resin composition further contains other resins, inorganic fillers, additives, and the like, these may be mixed in the above step (1) or may be mixed in step (2), in the production method I. Also, in the above production method II, they may be mixed in any step.

In step (1b) and (2b) of the production method II, the mixture (first premixture) obtained in step (1a) and (2a), the compound (B), and another part of (C) the organic peroxide are mixed. The first premixture may be once taken out as a pellet. The polyolefin resin composition in which the polyolefin resin (preferably a propylene-based resin and an ethylene-based copolymer) is uniformly dispersed can be obtained by adding the compound (B) and another part of the organic peroxide with, if necessary, an additive to the first premixture, and further melt-kneading.

In a preferred aspect of the present invention, the mixing of (B) the compound having at least one (meth)acrylate group, and one hydrogen bonding group or one (meth)acrylate group in the above step (1) and step (1b) is carried out by using a masterbatch at least containing the compound and a resin (preferably at least one resin selected from the group consisting of a polyolefin-based resin and an ethylene-based copolymer, and more preferably an ethylene-based copolymer).

In dry-mixing, powders may be mixed by putting the powders in a bag and swirling by hand, or a Henschel mixer, a sand mill, an edge runner, a Taninaka-type crusher, and the like may be used. In melt-kneading, a Banbury Mixer, a single-screw extruder, a twin-screw extruder, and the like may be used, and two or more extruders may be used in combination. From the viewpoint of easily increasing productivity, a twin-screw extruder is preferably used in melt-kneading. The temperature during melt-kneading (e.g., the set temperature of an extruder) is 150°C or more, preferably 160 to 300°C, more preferably 170 to 250°C, and further preferably 190°C to 230°C from the viewpoint of easily reducing the color tone derived from the polyolefin resin composition itself.

### <Molded Body of Polyolefin Resin Composition>

Molded bodies can be produced by molding the polyolefin resin composition of the present invention by a known molding method. Examples of the molding method include injection molding, press molding, vacuum molding, vacuum press molding, compressed air molding, foam molding, and extrusion molding, and injection molded bodies, press molded bodies, vacuum molded bodies, vacuum press molded bodies, compressed air molded bodies, foam molded bodies, and extrusion molded bodies can be obtained by these molding methods, respectively. The preferred molding method is injection molding, from the viewpoint of capable of making various molded articles and versatility. Examples of injection molding include common injection molding, injection foam molding, supercritical injection foam molding, ultra-high speed injection molding, injection compression molding, injection press molding, gas-assisted injection molding, sandwich molding, sandwich foam molding, and insert and outsert molding.

For example, the applications of the molded bodies obtained by molding the polyolefin resin composition of the present invention by the above molding methods are not particularly limited, and are preferably automobile interior parts such as bumpers, door trims, pillars, instrument panels, consoles, rocker panels, armrests, door panels, and spare tire covers.

### Examples

Hereinafter, the present invention will be described further in detail by way of Examples. Unless otherwise indicated, "%" and "part" in Examples mean % by mass and part by mass, respectively. First, measurement methods of physical properties are described.

### Measurement of Intrinsic Viscosity

The limiting viscosity (unit: dl/g) is a value measured using tetralin as a solvent by the following method at a temperature of 135°C.

Reduced viscosities were measured at three concentrations of 0.1 g/dl, 0.2 g/dl, and 0.5 g/dl using an Ubbelohde viscometer. The limiting viscosity can be determined by a calculation method described in "Kobunshi Yoeki, Kobunshi Jikkengaku 11 (Polymer Solution, Polymer Experiment 11)" (published by Kyoritsu Shuppan Co., Ltd., 1982) p. 491, i.e., by an extrapolation method in which reduced viscosity is plotted against concentration and the concentration is extrapolated in zero.

### Measurement of Melt Flow Rate

The melt flow rates (in the present specification, sometimes described as MFR) of the pellets of the polyolefin resin compositions obtained in Examples and Comparative Examples were measured at a temperature of 230°C and a load of 2.16 kg using a melt indexer (model L246-3537, manufactured by TECHNOL SEVEN CO., LTD.). The higher the numerical value of the melt flow rate, the more excellent the fluidity and the processability are.

### Izod Impact Strength (Izod, unit: kJ/m²)

The pellets of the polyolefin resin compositions obtained in Examples and Comparative Examples were injection molded under conditions of a mold temperature of 220°C and a mold cooling temperature of 50°C using an IS100 type injection molding machine manufactured by Toshiba Machine Co., Ltd. to mold Izod impact test specimens with a width of 12.7 mm, a length of 63.5 mm, and a thickness of 3.2 mm, and then a V notch (A type) with a dimension described in IS-K-7110 was formed on the Izod impact test specimens to produce test specimens.

The fabricated test specimens were allowed to stand at 23°C for 1 hour or more to condition and then subjected to Izod impact tests using a digital impact tester manufactured by Toyo Seiki Seisaku-sho, Ltd., thereby measuring the Izod impact strength.

### Measurement of Yellow Index

The pellets of the polyolefin resin compositions obtained in Examples and Comparative Examples were pressed under conditions of a temperature of 220°C using a press ("PEW-5040" manufactured by KANSAI ROLL Co., Ltd.) to give sheets with a thickness of 1 mm. The yellow index (yellowness, YI) of the obtained sheets was measured using a colorimeter ("CM-3500d" manufactured by KONICA MINOLTA JAPAN, INC.). A lower YI indicates that the change in color is more suppressed.

### Production Example 1: Production of Polyolefin Resin (A-1)

A polymerization catalyst was produced according to the method disclosed in Example 1 of Japanese Patent Laid-Open No. 2004-182981, and using the polymerization catalyst, a polyolefin resin (A-1) containing 79 parts by mass of a propylene homopolymer component as a propylene-based resin and 21 parts by mass of an ethylene-propylene random copolymer component as an ethylene-based copolymer was produced by a liquid phase-gas phase polymerization method. The melt flow rate (230°C, 2.16 kg load) of the obtained polyolefin resin was 25 g/10 minutes. The intrinsic viscosity of the propylene homopolymer component (P part, propylene-based resin pp1) contained in the polyolefin resin (A-1) was 1.1 dl/g. The intrinsic viscosity of the ethylene-propylene random copolymer component (EP part, ethylene-based copolymer ep1) was 2.8 dl/g, and the content of the monomer unit derived from ethylene contained in the ethylene-based copolymer ep1 was 33% by mass, based on the total amount of the ethylene-based copolymer.

### Production Example 2: Production of Polyolefin Resin (A-2)

A polymerization catalyst was produced according to the method disclosed in Example 1 of Japanese Patent Laid-Open No. 2004-182981, and using the polymerization catalyst, a polyolefin resin (A-2) containing 89 parts by mass of a propylene homopolymer component as a propylene-based resin and 11 parts by mass of an ethylene-propylene random copolymer component as an ethylene-based copolymer was produced by a liquid phase-gas phase polymerization method. The melt flow rate (230°C, 2.16 kg load) of the obtained polyolefin resin was 98 g/10 minutes. The intrinsic viscosity of the propylene homopolymer component (P part, propylene-based resin pp2) contained in the polyolefin resin (A-2) was 0.79 dl/g. The intrinsic viscosity of the ethylene-propylene random copolymer component (EP part, ethylene-based copolymer ep2) was 7.0 dl/g, and the content of the monomer unit derived from ethylene contained in the ethylene-based copolymer ep2 was 32% by mass, based on the total amount of the ethylene-based copolymer.

### Production Example 3: Production of Masterbatch containing Organic Peroxide (C-1)

Into 90 parts by mass of a propylene homopolymer, 10 parts by mass of an organic peroxide (C-1) (2,5-dimethyl-2,5-di(t-butylperoxy)hexane) was impregnated at room temperature to produce a polypropylene powder containing 10% by mass of the organic peroxide (C-1).

### Masterbatch containing Organic Peroxide (C-2)

A masterbatch containing 8% by mass of an organic peroxide (C-2) was used.

### Example 1

100 parts by mass of the polyolefin resin (A-1) obtained in Production Example 1, 1 part by mass of the masterbatch obtained in Production Example 3 (containing 0.1 parts by mass of the organic peroxide (C-1)), and 0.14 parts by mass of Sumilizer GM manufactured by SUMITOMO CHEMICAL COMPANY, LIMITED. as a compound (B-1) were weighed and uniformly mixed. Thereafter, the mixture was fed from the raw material feeding port located on the most upstream side of a 30 mm diameter twin-screw extrusion molding machine (NAS30 type extruder manufactured by Nakatani Machinery Ltd.) and melt-kneaded under conditions of a cylinder temperature of 220°C, a discharge amount of 2.4 kg/hour, and a screw rotation speed of 70 rpm to uniformly disperse the raw materials, thereby obtaining a pellet of the polyolefin resin composition.

### Examples 2 to 4 and Comparative Examples 1 and 2

Pellets of the polyolefin resin compositions were obtained in the same manner as in Example 1, except that the content of the organic peroxide (C-1) or the contents of the compound (B-1) and the organic peroxide were changed as described in Table 1.

### Examples 5 and 6

Pellets of the polyolefin resin compositions were obtained in the same manner as in Example 1, except that Sumilizer GS manufactured by SUMITOMO CHEMICAL COMPANY, LIMITED. was used as a compound (B-2) instead of the compound (B-1) and the content of the compound (B-2) was changed as described in Table 1.

### Comparative Example 3

A pellet of the polyolefin resin composition was obtained in the same manner as in Example 1, except that fulvene was used as a compound (B-3) instead of the compound (B-1) and the content of the compound (B-3) was changed as described in Table 1.

### Example 7

77 parts by mass of the polyolefin resin (A-2) obtained in Production Example 2, 23 parts by mass of an ethylene-butene-1 copolymer (eb1, "Excellen FX555" manufactured by SUMITOMO CHEMICAL COMPANY, LIMITED., the content of the monomer unit derived from ethylene: 76% by mass, the content of the monomer unit derived from butene-1: 24% by mass), and the masterbatch containing the organic peroxide (C-2) were weighed in such an amount that the amount of the organic peroxide (C-2) could be the amount described in Table 1, and uniformly mixed. Thereafter, the mixture was fed from the raw material feeding port located on the most upstream side of a twin-screw molding machine, TEX44αII manufactured by The Japan Steel Works, LTD., and melt-kneaded under conditions of a cylinder temperature of 230°C, a discharge amount of 50 kg/hour, and a screw rotation speed of 200 rpm to uniformly disperse the raw materials, thereby obtaining a pellet of the resin composition precursor (first premixture). With respect to 100 parts by mass of the thus obtained pellet, 1.64 parts by mass of the compound (B-1) and the masterbatch containing the organic peroxide (C-1) obtained in Production Example 3 were weighed in such an amount that the amount of the organic peroxide (C-1) could be the amount shown in Table 1, and uniformly mixed in advance. Thereafter, the mixture was fed from the raw material feeding port located on the most upstream side of the twin-screw molding machine TEX44αII manufactured by The Japan Steel Works, LTD., and melt-kneaded under conditions of a cylinder temperature of 230°C, a discharge amount of 50 kg/hour, and a screw rotation speed of 200 rpm to uniformly disperse the raw materials, thereby obtaining a pellet of the polyolefin resin composition.

### Comparative Example 4

A pellet of the polyolefin resin composition was obtained in the same manner as in Example 7, except that the organic peroxide (C-1) and the compound (B-1) were not added.

Using the pellets of the resin compositions obtained in Examples 1 to 7 and Comparative Examples 1 to 4, the melt flow rate, Izod impact strength, and yellow index were measured according to the above methods. The results obtained are shown in Table 1 with the composition of each resin composition. The polyolefin resin (A-1) contains 79 parts by mass of a propylene homopolymer component and 21 parts by mass of an ethylene-propylene random copolymer component, as described above, and the polyolefin resin (A-2) contains 89 parts by mass of a propylene homopolymer component and 11 parts by mass of an ethylene-propylene random copolymer component, as described above. In addition, B-3 in Table 1 is fulvene and this is not the compound corresponding to the compound (B) in the present invention.

**[Table 1]**

| | | (A) Polyolefin resin | | | (B) Compound | | | (C) Organic peroxide | | Mole ratio (B/C) | MFR [g/10 min] | Izod [kJ/m²] | YI |
|---|---|---|---|---|---|---|---|---|---|---|---|---|---|
| | | A-1 | A-2 | eb1 | B-1 | B-2 | B-3 | C-1 | C-2 | | | | |
| Example | 1 | 100 | - | - | 0.14 | - | - | 0.1 | - | 1.0 | 77 | 15 | 0 |
| | 2 | 100 | - | - | 0.27 | - | - | 0.1 | - | 2.0 | 49 | 16 | 0 |
| | 3 | 100 | - | - | 0.54 | - | - | 0.2 | - | 2.0 | 68 | 16 | 5 |
| | 4 | 100 | - | - | 0.82 | - | - | 0.3 | - | 2.0 | 48 | 17 | 10 |
| | 5 | 100 | - | - | - | 0.19 | - | 0.1 | - | 1.0 | 87 | 12 | 5 |
| | 6 | 100 | - | - | - | 0.38 | - | 0.1 | - | 2.0 | 54 | 13 | 0 |
| | 7 | - | 77 | 23 | 1.64 | - | - | 0.6 | 0.06 | 2.0 | 88 | 32 | 4 |
| Compar ative Example | 1 | 100 | - | - | - | - | - | - | - | - | 31 | 14 | 0 |
| | 2 | 100 | - | - | - | - | - | 0.1 | - | 0.0 | 187 | 5 | -0.5 |
| | 3 | 100 | - | - | - | - | 0.16 | 0.1 | - | 2.0 | 53 | 16 | 28 |
| | 4 | - | 77 | 23 | - | - | - | - | 0.06 | - | 82 | 11 | 0 |

In one aspect using the above compound (Ba) having one (meth)acrylate group and at least one hydrogen bonding group, it was confirmed that the polyolefin resin compositions of the present invention of Examples 1 to 7 containing at least: (A) 100 parts by mass of a polyolefin resin, 0.01 to 5 parts by mass of the compound (Ba), and (C) 0.01 to 5 parts by mass of an organic peroxide had a high MFR and excellent fluidity, and the molded bodies produced using the resin compositions had excellent impact resistance and a low yellowness YI. In contrast, the resin composition of Comparative Example 1 which contains neither compound (B) (especially, compound (Ba)) nor organic peroxide had insufficient fluidity. Further, the resin compositions of Comparative Examples 2 and 4 which contain an organic peroxide but contain no compound (B) (especially, compound (Ba)) resulted in high fluidity but low impact resistance. In addition, the resin composition of Comparative Example 3 containing fulvene (B-3) which does not correspond to the compound (B) (especially, compound (Ba)) of the present invention resulted in high YI of the molded body.

### Synthesis of Compound (B-4)

After a four neck flask was charged with 100 g (0.82 mol) of 2,4-dimethylphenol, 12.9 g (0.14 mol) of trioxane, 48.0 g of xylene, 89.8 g of water, and 3.0 g of 98% sulfuric acid, the flask was allowed to warm to 120°C and held under reflux conditions for 16 hours. Thereafter, the aqueous phase was removed by liquid separation, and then the precipitate was filtered, and the filtrate was washed with hexane. Thereafter, the precipitate was dissolved in 300 ml of toluene, and then the toluene layer was separated and washed with 100 ml of water three times, and the toluene layer was concentrated and dried to give 43 g of 6,6'-methylenebis(2,4-dimethylphenol) in 41% yield with a purity of 97%.

After a four neck flask was charged with 42.9 g of the obtained 6,6'-methylenebis(2,4-dimethylphenol), 43.2 g of xylene, 43.2 g of triethylamine, and 12.0 g of acrylic acid and allowed to warm to 75°C, 19.0 g of phosphoryl chloride was added dropwise over 2 hours thereto, and then the flask was heated and held for 30 minutes. Thereafter, the mixture was separated and washed with 130 g of water three times, and a crude product obtained by concentrating and drying the xylene phase was purified by a silica gel column to give 6.7 g of a compound represented by the following formula (B-4) in 13% yield with a purity of 98%.
[Formula 15]

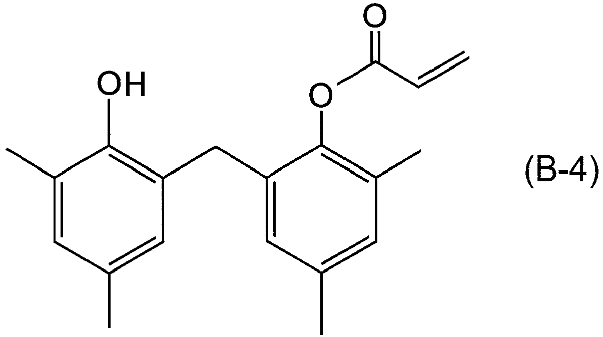

The measurement results of ¹H-NMR and MS of the compound (B-4) are as follows.

¹H-NMR (CDCl₃, 400 MHz): δ 2.12, 2.17, 2.21, 2.24 (s, 12H, Me), δ 3.69 (s, 2H, Ar-CH₂-Ar), δ 6.05 (dd, J=10.4, 1.2 Hz, 1H, CH=CH₂), δ 6.66 (dd, J=17.2, 1.2 Hz, 1H, CH=CH₂), δ 6.37 (dd, J=17.4, 10.4 Hz, 1H, CH=CH₂), δ 6.74, 6.79, 6.84, 6.91 (s, 4H, Ar)

MS (ESI) m/z (rel intensity) 309.5 ([M-H⁻], 100) [0101] Synthesis of Compound (B-5)

After a four neck flask was charged with 30 g (0.15 mol) of 2,4-ditertiarybutylphenol, 2.3 g (0.025 mol) of trioxane, 14.4 g of xylene, 26.4 g of water, and 1.0 g of 98% sulfuric acid, the flask was allowed to warm to 120°C and held under reflux conditions for 12 hours. Thereafter, the aqueous phase was removed by liquid separation, and then the xylene phase was washed with 100 ml of brine three times. Thereafter, the concentrated and dried product was dissolved in 60 g of hexane by heating and recrystallized, and the precipitate was filtered and dried to give 20 g of 6,6'-methylenebis(2,4-ditertiarybutylphenol) in 57% yield with a purity of 99.7%.

After a four neck flask was charged with 19.7 g of the obtained 6,6'-methylenebis(2,4-ditertiarybutylphenol), 20.0 g of xylene, 20.0 g of triethylamine, and 3.3 g of acrylic acid, and allowed to warm to 75°C, 8.8 g of phosphoryl chloride was added dropwise over 1.5 hours, and then heated and held for 1 hour. Thereafter, the mixture was separated and washed with 45 g of water three times, and then the crude product obtained by concentrating and drying the xylene phase was purified by a silica gel column to give 8.9 g of a compound represented by the following formula (B-5) in 40% yield with a purity of 98%.

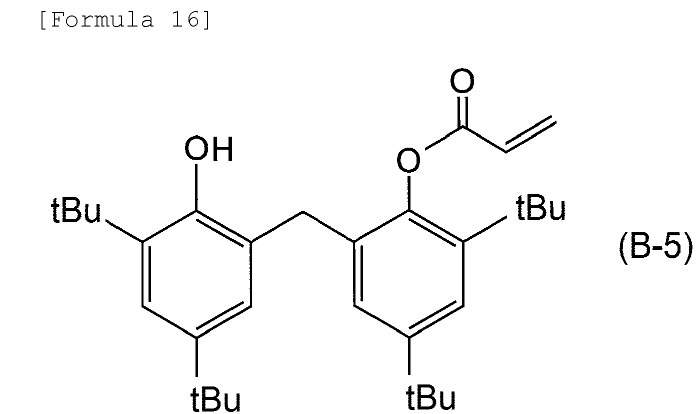

The measurement results of ¹H-NMR and MS of the compound (B-5) are as follows.

¹H-NMR (CDCl₃, 400 MHz): δ 1.15, 1.30, 1.36, 1.37 (s, 36H, t-Bu), δ 3.70 (d, J=3.9 Hz, 2H, Ar-CH₂-Ar), δ 5.15 (d, J=0.5 Hz, 1H, OH), δ 6.09 (dd, J=10.5, 1.2 Hz, 1H, CH=CH₂), δ 6.67 (dd, J=17.3, 1.2 Hz, 1H, CH=CH₂), δ 6.41 (dd, J=17.2, 10.4 Hz, 1H, CH=CH₂), δ 6.76, 6.98, 7.23, 7.30 (d, J=2.5, 2.4, 2.4, 2.2 Hz, 4H, Ar)

MS (ESI) m/z (rel intensity) 477.5 ([M-H⁻], 100) [0103] Synthesis of Compound (B-6)

A four neck flask was charged with 306 g (1.86 mol) of 2-tertiary-butyl-p-cresol, 169 ml of xylene, 6.12 g of PELEX NBL (manufactured by Kao Corporation), and 313 g of 2.3% sulfuric acid, and allowed to warm to 50°C. Thereafter, 43.9 g (326 mmol) of paraldehyde was added dropwise thereto over 2 hours. After dropwise addition, the flask was allowed to warm to 95°C and the temperature was kept under heating and reflux conditions for 5 hours. Thereafter, the mixture was allowed to cool to 60°C, then 0.544 g (4.03 mmol) of paraldehyde was added thereto, the mixture was allowed to warm to 95°C again, and the temperature was kept under reflux conditions for 15 hours. Thereafter, the mixture was cooled to 0°C, and the precipitate was filtered and washed with water to give a crude product. The crude product was dissolved in 350 ml of hexane by heating, the mixture was cooled to 0°C while stirring, and the precipitate was washed with a small amount of hexane to give 133 g of 6,6'-methylenebis(2-tertiarybutyl-4methylphenol) with a purity of 99%.

After a four neck flask was charged with 66.5 g (188 mmol) of the obtained 6,6'-methylenebis(2-tertiarybutyl-4methylphenol), 66.5 g of xylene, 39.9 g (394 mmol) of triethylamine, and 13.5 g (187 mmol) of acrylic acid, and allowed to warm to 55°C, 17.9 g (117 mmol) of phosphoryl chloride was added dropwise over 1 hour. Thereafter, the mixture was allowed to cool to room temperature, and then water was added to quench the reaction, and the precipitated crystal was filtered and washed with water to give a crude product. The crude product was washed with methanol and dried to give 65.1 g of a compound represented by formula (B-6) in 85% yield with a purity of 99%.

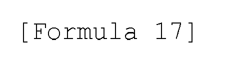

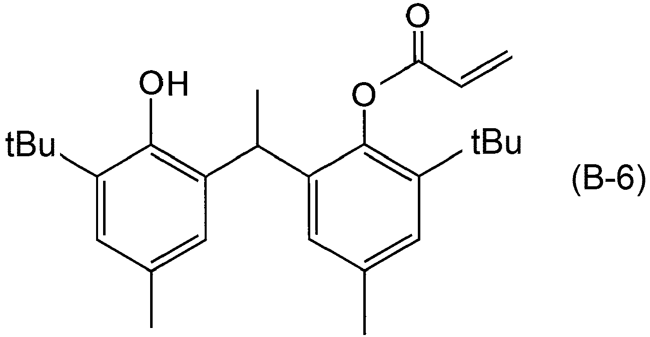

The measurement results of ¹H-NMR and MS of the compound (B-6) are as follows.

¹H-NMR (CDCl₃, 400 MHz): δ 1.35, 1.36 (s, 18H, t-Bu), δ 1.46 (d, J=7.1 Hz, 3H, Ar-CH (-CH₃)-Ar), δ 2.15, 2.35 (s, 6H, Ar-CH₃), δ 3.85 (q, J=6.8 Hz, 1H, Ar-CH (-CH₃)-Ar), δ 6.16, 6.78 (d, J=1.0 Hz, 2H, CH=CH₂), δ 6.45 (dd, J=6.1, 4.4 Hz, 1H, CH=CH₂), δ 6.4-7.1 (s, 4H, Ar)

MS (ESI) m/z (rel intensity) 407.5 ([M-H]⁻, 100) [0105] Synthesis of Compound (B-7)

A four neck flask was charged with 230 g (1.88 mol) of 2,4-dimethylphenol, 110 g (1.20 mol) of xylene, 44.8 g (0.339 mol) of paraldehyde, 230 g of water, 7.3 g (0.074 mol) of 98% sulfuric acid, and 2.54 g of PELEX NBL (manufactured by Kao Corporation), and allowed to warm to 120°C. Thereafter, the temperature was kept under reflux conditions for 20 hours, and then 10.9 g (0.082 mol) of paraldehyde was added, and heating at reflux was continued for 8 hours again. After 28 hours of the total temperature keeping time at reflux, after reflux conditions were additionally held for 8 hours, 4.35 g (0.033 mol) of paraldehyde was added again, and further, the temperature was continued to be kept for 13 hours at reflux. Thereafter, 151 g of xylene was added and the temperature was adjusted to 70°C, and then the mixture was allowed to cool to room temperature while stirring. Thereafter, the precipitate was filtered, the filtrate was washed with 500 ml of water three times, and then dried. 43.9 g (326 mmol) of paraldehyde was added dropwise over 2 hours. After dropwise addition, the mixture was allowed to warm to 95°C and the temperature was kept for 5 hours, while being heated to reflux. Thereafter, the mixture was allowed to cool to 60°C, then 0.544 g (4.03 mmol) of paraldehyde was added thereto, the mixture was allowed to warm to 95°C again, and the temperature was kept under reflux conditions for 15 hours. Thereafter, 151 g of xylene was added and the mixture was allowed to cool to room temperature. The precipitate was filtered, and the filtrate was washed with 500 ml of water three times. The obtained filtrate was dried under vacuum and then dissolved in 789 g of toluene by heating, 641 g of hexane was added thereto in a state where the internal temperature was adjusted to 60°C, and the mixture was allowed to cool to room temperature while stirring. The precipitated solid was filtered, and the filtrate was washed with 800 ml of hexane three times and dried under vacuum to give 146 g of 1,1-bis(2-hydroxy-3,5-dimethylphenyl)ethane as the objective product in 57% yield with a purity of 99%.

After a four neck flask was charged with 145 g (0.537 mol) of the obtained 1,1-bis(2-hydroxy-3,5-dimethylphenyl)ethane, 146 g of xylene, 116 g of triethylamine, and 38.4 g (0.533 mmol) of acrylic acid, and allowed to warm to 63°C, 50.9 g (0.322 mol) of phosphoryl chloride was added dropwise over 1 hour 15 minutes. The internal temperature at this time was 75 to 78°C. Thereafter, the temperature was kept at 72°C for 1 hour by heating, the mixture was allowed to cool to 60°C and then to room temperature, and 440 ml of water and 500 ml of toluene were added. Further, after 750 ml of toluene and a small amount of saline were added, the mixture was subjected to liquid separation. Thereafter, the toluene phase was washed with 450 ml of saline, 438 g of 4.8% aqueous sodium carbonate solution, and 450 ml of saline, in the order presented. Thereafter, the toluene phase was dried over anhydrous sodium sulfate, and the solvent was distilled off under reduced pressure. The obtained residue was purified by column chromatography to give 64.6 g of a compound represented by the following formula (B-7) in 37% yield with a purity of 96%.

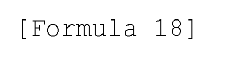

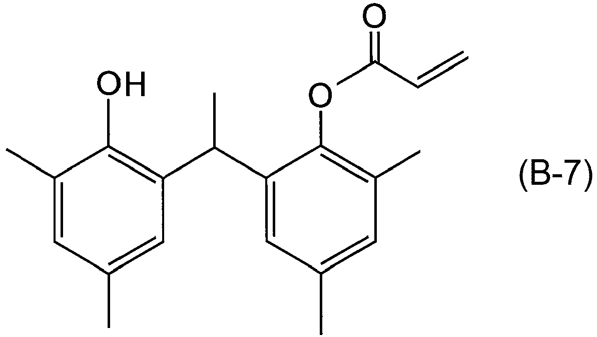

The measurement results of ¹H-NMR and MS of the compound (B-7) are as follows.

¹H-NMR (CDCl₃, 400 MHz): δ 1.50 (d, J=7.4 Hz, 3H, Ar-CH (-CH₃)-Ar), δ 2.12, 2.16, 2.19, 2.30 (s, 12H, Ar-CH₃), δ 4.04 (s, 1H, Ar-CH (-CH₃)-Ar), δ 6.08, 6.69 (d, J=10.8, 17.1 Hz, 2H, CH=CH₂), δ 6.40 (dd, J=25.6, 10.4 Hz, 1H, CH=CH₂), δ 6.6-7.0 (s, 4H, Ar)

MS (ESI) m/z (rel intensity) 323.5 ([M-H]⁻, 100) [0107] Synthesis of Compound (B-8)

A four neck flask was charged with 102 g (0.49 mol) of 2,4-ditertiarybutylphenol, 56 ml of xylene, 11.8 g (0.089 mol) of paraldehyde, 102 ml of water, 1.9 g (0.074 mol) of 98% sulfuric acid, and 1.0 g of PELEX NBL (manufactured by Kao Corporation), and allowed to warm to 92°C. Thereafter, the temperature was kept for 5 hours, then 5.9 g (0.044 mol) of paraldehyde was added, and heating and stirring were continued overnight. After 56 ml of xylene was added, the mixture was allowed to cool to 50°C, and then a seed crystal was added, and the mixture was further allowed to cool to 25°C. The precipitated solid was washed with water and a small amount of hexane and dried to give 64.9 g of 1,1-bis(2-hydroxy-3,5-ditertiarybutylphenyl)ethane as the objective product. The same operation was carried out twice.

After a four neck flask was charged with 73.4 g (0.167 mol) of the obtained 1,1-bis(2-hydroxy-3,5-ditertiarybutylphenyl)ethane, 86 ml of xylene, 49.7 g (0.356 mol) of triethylamine, and 11.9 g (0.166 mol) of acrylic acid, and allowed to warm to 80°C, 9.7 ml (0.104 mol) of phosphoryl chloride was added dropwise over 50 minutes. After completion of the reaction, the internal temperature was allowed to cool to 45°C, and then 150 ml of water was added dropwise and the mixture was extracted with toluene. After Celite filtration, the filtrate was washed with water, dried, and concentrated. The obtained solid was washed with methanol. The residue on the filter after Celite filtration was extracted with chloroform, dried over sodium sulfate, concentrated, and mixed with the solid obtained from the filtrate after Celite filtration, and then the mixture was washed with methanol again and dried to give 76.6 g of a compound represented by the following formula (B-8) in 93% yield.

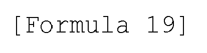

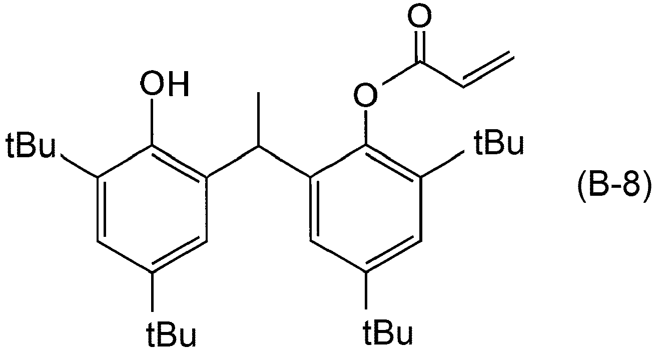

The measurement results of ¹H-NMR and MS of the compound (B-8) are as follows.

¹H-NMR (CDCl₃, 400 MHz): δ 1.08, 1.35, 1.36, 1.37 (s, 36H, t-Bu), δ 1.51 (d, J=7.1 Hz, 3H, Ar-CH (-CH₃)-Ar), δ 3.91 (q, J=13.9, 6.9 Hz, 1H, Ar-CH (-CH₃)-Ar), δ 6.13, 6.16 (dd, J=10.5, 1.0 Hz, 1H, CH=CH₂), δ 6.48 (dd, J=17.4, 10.5 Hz, 1H, CH=CH₂), δ 6.76 (dd, J=17.2, 0.9 Hz, 1H, CH=CH₂), δ 6.5-7.3 (s, 4H, Ar)

MS (ESI) m/z (rel intensity) 491.5 ([M-H]⁻, 100) [0109] Synthesis of Compound (B-9)

A four neck flask was charged with 50.0 g (0.14 mol) of 4,4-thiobis(2-tertiarybutyl-5-methylphenol) (WX-R manufactured by SUMITOMO CHEMICAL COMPANY, LIMITED), 100 g of dehydrated toluene, and 141 g (1.4 mol) of triethylamine, and the mixture was stirred and dissolved at room temperature. Thereafter, 51.5 g (0.56 mol) of acryloyl chloride was added dropwise over 8 hours. After termination of the reaction, the toluene phase was washed and separated with water three times, the toluene phase was concentrated and dried. The obtained concentrated product was purified by a silica gel column to give 12.2 g of a compound represented by the following formula (B-9) as the objective product in 20% yield with a purity of 99%.

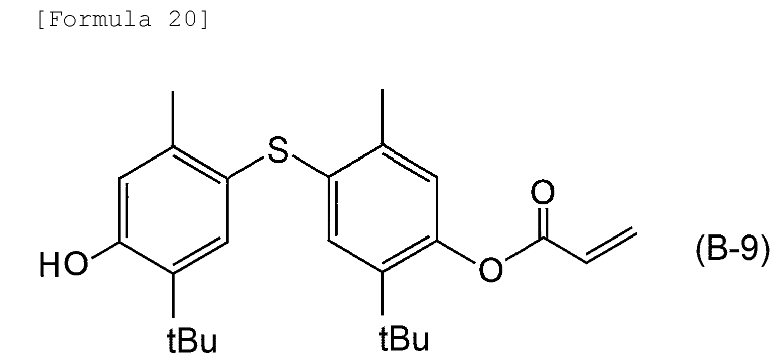

The measurement results of ¹H-NMR and MS of the compound (B-9) are as follows.

¹H-NMR (CDCl₃, 400 MHz): δ 1.14, 1.35 (s, 18H, t-Bu), δ 2.27, 2.33 (d, J=0.5 Hz, 6H, Me), δ 4.93 (s, 1H, OH), δ 6.03 (dd, J=10.5, 1.2 Hz, 1H, CH=CH₂), δ 6.34 (dd, J=17.3, 10.3 Hz, 1H, CH=CH₂), δ 6.60 (dd, J=17.4, 1.2 Hz, 1H, CH=CH₂), δ 6.6-7.3 (s, 4H, Ar)

### Examples 8 to 13

Pellets of the polyolefin resin compositions were obtained in the same manner as in Example 1 except that the compound (B-1) was changed, as described in Table 1, to the compounds (B-4) to (B-9) obtained as described above. Thereafter, the melt flow rate, Izod impact strength, and yellow index were measured using the pellets. The obtained results are shown in Table 2 with the composition of each resin composition.

**[Table 2]**

| | | (A) Polyolefin resin | (B) Compound | | | | | | (C) Organic peroxide | Mole ratio(B/C) | MFR [g/10 min] | Izod [kJ/m²] | YI |
|---|---|---|---|---|---|---|---|---|---|---|---|---|---|
| | | A-1 | B-4 | B-5 | B-6 | B-7 | B-8 | B-9 | C-1 | | | | |
| Example | 8 | 100 | 0.21 | - | - | - | - | - | 0.1 | 2 | 56 | 15 | 3 |
| | 9 | 100 | - | 0.33 | - | - | - | - | 0.1 | 2 | 52 | 17 | 8 |
| | 10 | 100 | - | - | 0.28 | - | - | - | 0.1 | 2 | 48 | 16 | 7 |
| | 11 | 100 | - | - | - | 0.22 | - | - | 0.1 | 2 | 44 | 19 | 4 |
| | 12 | 100 | - | - | - | - | 0.34 | - | 0.1 | 2 | 54 | 15 | 4 |
| | 13 | 100 | - | - | - | - | - | 0.28 | 0.1 | 2 | 90 | 14 | 6 |

In one aspect containing the compound (B), it was confirmed that the polyolefin resin compositions of the present invention of Examples 8 to 13 containing at least: (A) 100 parts by mass of a polyolefin resin, (B) 0.01 to 5 parts by mass of the compound having at least one (meth)acrylate group, and one hydrogen bonding group or one (meth)acrylate group, and (C) 0.01 to 5 parts by mass of an organic peroxide had a high MFR and excellent fluidity and the molded bodies produced using the resin compositions had excellent impact resistance and a low yellowness YI.

### Example 14: Production of Masterbatch

To 73 parts by mass of an ethylene/1-octene copolymer (ENGAGE8842 available from The Dow Chemical Company), 27 parts by mass of Sumilizer GM manufactured by SUMITOMO CHEMICAL COMPANY, LIMITED. was dry-blended as the compound (B-1), 4 parts by mass of the organic peroxide (C-1) (2,5-dimethyl-2,5-di(t-butylperoxy)hexane) was impregnated thereinto, and the mixture was fed from the raw material feeding port located on the most upstream side of a 30 mm diameter twin-screw extrusion molding machine (NAS30 type extruder manufactured by Nakatani Machinery Ltd.) and extruded at a cylinder temperature of 70°C to give a masterbatch.

Evaluation of Masterbatch 98 parts by mass of the polyolefin resin (A-1) obtained in Production Example 1 was mixed with 0.05 parts of calcium stearate, 0.05 parts of Irganox 1010, and 0.1 parts of Irgafos 168, and 1.8 parts by mass of a polypropylene powder containing 10% by mass of the organic peroxide (C-1) and 3.1 parts by mass of the masterbatch obtained in Example 14 were added thereto. After dry-blending, the mixture was fed from the raw material feeding port located on the most upstream side of a 30 mm diameter twin-screw extrusion molding machine (NAS30 type extruder manufactured by Nakatani Machinery Ltd.) and melt-kneaded under conditions of a cylinder temperature of 220°C, a discharge amount of 2.4 kg/hour, and a screw rotation speed of 70 rpm to uniformly disperse the raw materials, thereby obtaining a pellet of the polyolefin resin composition. The melt flow rate was measured using the pellet of the resin composition and the Izod impact strength and yellow index were measured using the injection molded products, according to the above methods. The obtained results are shown in Table 3.

**[Table 3]**

| Example | A-1 (phr) | Masterbatch (phr) | C-1 (phr) | MFR (g/10 min) | Izod (kJ/m²) | YI |
|---|---|---|---|---|---|---|
| 14 | 98 | 3.1 | 1.8 | 78 | 19 | 4 |

## Claims

1. A polyolefin resin composition comprising at least:
(A) 100 parts by mass of a polyolefin resin;
(B) 0.01 to 5 parts by mass of a compound having at least one (meth)acrylate group, and one hydrogen bonding group or one (meth)acrylate group; and
(C) 0.01 to 5 parts by mass of an organic peroxide.

2. The polyolefin resin composition according to claim 1, wherein (B) the compound having at least one (meth)acrylate group, and one hydrogen bonding group or one (meth)acrylate group is represented by formula (I):
[Formula 1] 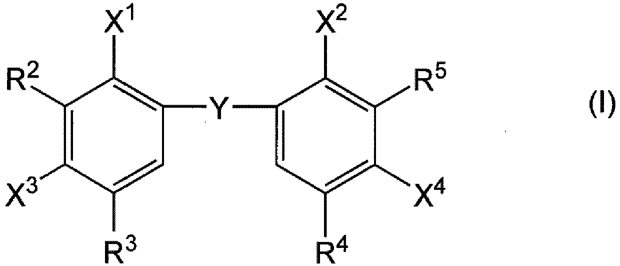 wherein
X¹ and X² each independently represent a hydrogen bonding group or a group represented by -OC(=O)CR¹=CH₂ and X³ and X⁴ each independently represent a hydrogen atom or a linear or branched alkyl group having 1 to 9 carbon atoms, or X¹ and X² each independently represent a hydrogen atom or a linear or branched alkyl group having 1 to 9 carbon atoms and X³ and X⁴ each independently represent a hydrogen bonding group or a group represented by -OC(=O)CR¹=CH₂, provided that at least one of X¹ to X⁴ represents a group represented by -OC(=O)CR¹=CH₂, and R¹ represents a hydrogen atom or a methyl group;
Y represents a single bond, a sulfide bond, or a linear or branched alkylene group having 1 to 9 carbon atoms; and
R² and R⁵ each independently represent a hydrogen atom or a linear or branched alkyl group having 1 to 9 carbon atoms, and R³ and R⁴ each independently represent a linear or branched alkyl group having 1 to 9 carbon atoms.

3. The polyolefin resin composition according to claim 2, wherein in formula (I), X¹ represents a hydrogen bonding group or a group represented by -OC(=O)CR¹=CH₂; X² represents a group represented by -OC(=O)CR¹=CH₂; X³ and X⁴ represent a hydrogen atom; Y represents a single bond or a linear or branched alkylene group having 1 to 9 carbon atoms; R¹ represents a hydrogen atom or a methyl group; and R² to R⁵ each independently represent a linear or branched alkyl group having 1 to 9 carbon atoms.

4. The polyolefin resin composition according to claim 2, wherein in formula (I), X¹ and X² represent a linear or branched alkyl group having 1 to 9 carbon atoms; X³ represents a hydrogen bonding group or a group represented by -OC(=O)CR¹=CH₂; X⁴ represents a group represented by -OC(=O)CR¹=CH₂; Y represents a sulfide bond; R¹ represents a hydrogen atom or a methyl group; R² and R⁵ represent a hydrogen atom; and R³ and R⁴ each independently represent a linear or branched alkyl group having 1 to 9 carbon atoms.

5. The polyolefin resin composition according to claim 1, wherein (B) the compound having at least one (meth)acrylate group, and one hydrogen bonding group or one (meth)acrylate group is a compound having one (meth)acrylate group and at least one hydrogen bonding group.

6. The polyolefin resin composition according to claim 5, wherein (B) the compound having one (meth)acrylate group and at least one hydrogen bonding group is represented by formula (Ia):
[Formula 2] 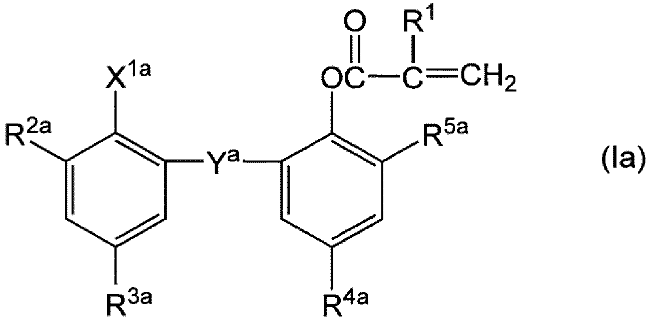 wherein
X^{1a} represents a hydrogen bonding group;
Y^{a} represents a single bond or a linear or branched alkylene group having 1 to 9 carbon atoms;
R¹ represents a hydrogen atom or a methyl group; and
R^{2a} to R^{5a} each independently represent a linear or branched alkyl group having 1 to 9 carbon atoms.

7. The polyolefin resin composition according to claim 6, wherein formula (I) is represented by formula (Ia'):
[Formula 3] 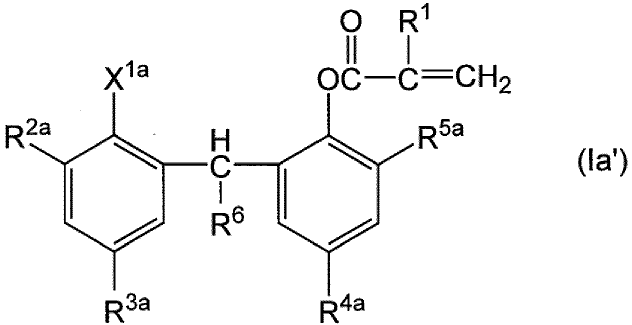 wherein X^{1a}, R¹ and R^{2a} to R^{5a} are as defined for X^{1a}, R¹ and R^{2a} to R^{5a} in formula (Ia), respectively; and R⁶ represents a hydrogen atom or a methyl group.

8. The polyolefin resin composition according to any one of claims 1 to 7, wherein the hydrogen bonding group is selected from the group consisting of a hydroxyl group, an amino group, a thiol group, a carboxyl group, a sulfonic acid group, and a phosphoric acid group.

9. The polyolefin resin composition according to claim 7, wherein in formula (Ia'), X^{1a} represents a hydroxyl group; R¹ represents a hydrogen atom; R^{2a} to R^{5a} represent a 1,1-dimethylpropyl group; and R⁶ represents a methyl group.

10. The polyolefin resin composition according to claim 7, wherein in formula (Ia'), X^{1a} represents a hydroxyl group; R¹ and R⁶ represent a hydrogen atom; R^{2a} and R^{5a} represent a t-butyl group; R^{3a} and R^{4a} represent a methyl group.

11. The polyolefin resin composition according to claim 2, wherein formula (I) is represented by formula (Id):
[Formula 4] 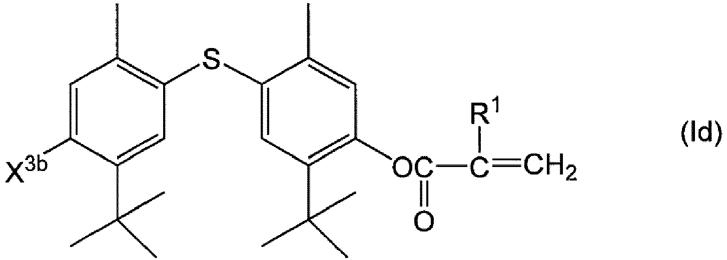 wherein X^{3b} represents a hydrogen bonding group or a group represented by -OC(=O)CR¹=CH₂; and R¹ represents a hydrogen atom.

12. The polyolefin resin composition according to any one of claims 1 to 11, wherein (C) the organic peroxide is selected from the group consisting of an alkyl peroxide compound, a diacyl peroxide compound, a peroxy ester compound, and a peroxycarbonate compound.

13. The polyolefin resin composition according to any one of claims 1 to 12, wherein a mass ratio of (B) the compound having at least one (meth)acrylate group, and one hydrogen bonding group or one (meth)acrylate group:(C) the organic peroxide is 1:10 to 10:1.

14. The polyolefin resin composition according to any one of claims 1 to 13, wherein (A) the polyolefin resin comprises a propylene-based resin.

15. The polyolefin resin composition according to claim 14, wherein the propylene-based resin comprises 80% by mass or more of a monomer unit derived from propylene, based on the total amount of the propylene-based resin.

16. The polyolefin resin composition according to claim 14 or 15, wherein (A) the polyolefin resin further comprises an ethylene-based copolymer.

17. The polyolefin resin composition according to claim 16, wherein the ethylene-based copolymer comprises 20% by mass or more of a monomer unit derived from ethylene, based on the total amount of the ethylene-based copolymer.

18. A method for producing the polyolefin resin composition according to any one of claims 1 to 17, comprising at least a step of mixing (A) a polyolefin resin, (B) a compound having at least one (meth)acrylate group, and one hydrogen bonding group or one (meth)acrylate group, and (C) an organic peroxide together by melt-kneading a mixture obtained by dry-mixing in advance.

19. The production method according to claim 18, comprising either step (1) of dry-mixing (A) the polyolefin resin, (B) the compound having at least one (meth)acrylate group, and one hydrogen bonding group or one (meth)acrylate group, and (C) the organic peroxide to give a mixture; and step (2) of melt-kneading the mixture obtained in step (1) to give the polyolefin resin composition, or
step (1a) of dry-mixing (A) the polyolefin resin and a part of (C) the organic peroxide to give a first dry mixture; step (2a) of melt-kneading the dry mixture obtained in step (1a) to give a first premixture; step (1b) of dry-mixing the first premixture obtained in step (2a), (B) the compound having at least one (meth)acrylate group, and one hydrogen bonding group or one (meth)acrylate group, and another part of (C) the organic peroxide to give a second dry mixture; and step (2b) of melt-kneading the dry mixture obtained in step (1b) to give the polyolefin resin composition.

20. The production method according to claim 18 or 19, wherein the mixing of (B) the compound having at least one (meth)acrylate group, and one hydrogen bonding group or one (meth)acrylate group in step (1) and step (1b) is carried out by using a masterbatch comprising at least the compound and at least one resin selected from the group consisting of a polyolefin-based resin and an ethylene-based copolymer.

21. A masterbatch comprising a compound having at least one (meth)acrylate group, and one hydrogen bonding group or one (meth)acrylate group, and at least one resin selected from the group consisting of a polyolefin-based resin and an ethylene-based copolymer.

22. The masterbatch according to claim 21, comprising 10 to 50 parts by mass of the compound having at least one (meth)acrylate group, and one hydrogen bonding group or one (meth)acrylate group with respect to 100 parts by mass of the polyolefin-based resin and the ethylene-based copolymer.

23. The masterbatch according to claim 20 or 21, further comprising an organic peroxide.

24. A compound represented by formula (Ic):
[Formula 5] 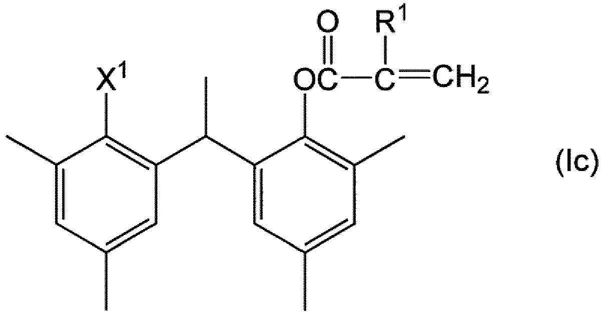 wherein X¹ represents a hydrogen bonding group or a group represented by -OC(=O)CR¹=CH₂; and R¹ represents a hydrogen atom.

25. A compound represented by formula (Id):
[Formula 6] 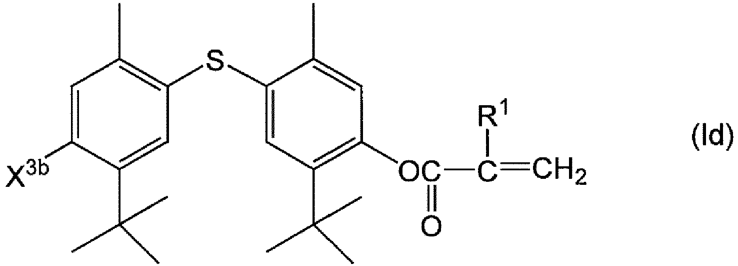 wherein X^{3b} represents a hydrogen bonding group or a group represented by -OC(=O)CR¹=CH₂; and R¹ represents a hydrogen atom.
